# EUROPEAN PATENT SPECIFICATION

(11) **EP 4 028 505 B1**
(45) Date of publication and mention of the grant of the patent: **14.06.2023**
(21) Application number: 21759749.1
(22) Date of filing: 25.02.2021
(51) Int. Cl.: C12N 5/00, C12N 5/077, C12N 5/074

(54) **A METHOD FOR PROVIDING A CARTILAGE IMPLANT WITH CHONDROCYTES**
VERFAHREN ZUR HERSTELLUNG EINES KNORPELIMPLANTATS MIT CHONDROZYTEN
PROCÉDÉ DE PRÉPARATION D'UN IMPLANT CARTILAGINEUX À CHONDROCYTES

(30) Priority: 28.02.2020 EP 20160158
(43) Date of publication of application: 20.07.2022
(73) Proprietor: Cline Scientific AB, 413 53 Mölndal (SE)
(72) Inventor: ANDREASSON, Linnea, 413 04 Göteborg (SE); HOLMQUIST, Niklas, 434 91 Kungsbacka (SE); SUNDH, Patrik, 423 41 Torslanda (SE); EVENBRATT, Hanne, 443 95 Stenkullen (SE); SIMONSSON, Stina, 431 69 Mölndal (SE)
(74) Representative: Ström & Gulliksson AB
(86) International application number: PCT/SE2021/050157
(87) International publication number: WO 2021/173066

(56) References cited:
- EP-A1- 2 608 896
- EP-A1- 3 064 577
- EP-A1- 3 260 536
- EP-A1- 3 446 722
- WO-A1-2012/025576
- WO-A1-2013/010045
- WO-A1-2018/174403
- JP-A- 2019 136 042
- US-A1- 2019 111 086
- US-B2- 9 089 598
- US-B2- 9 452 238
- Linnea Andreasson: "Effects on Chondrocyte Derived Induced Pluripotent Stem Cells Adhered to Nano Gradients Functionalized with Transforming Growth Factor Beta-1, -3 and Growth Differentiation Factor 5 Master Thesis in Biotechnology", , 1 June 2018 (2018-06-01), XP055707940, Retrieved from the Internet: URL:http://publications.lib.chalmers.se/re cords/fulltext/256174/256174.pdf [retrieved on 2020-06-23]
- ANDREASSON L: "Effects on Chondrocyte Derived Induced Pluripotent Stem Cells Adhered to Nano Gradients Functionalized with Transforming Growth Factor Beta-1, -3 and Growth Differentiation Factor 5", Dissertation, 1 June 2018 (2018-06-01), pages 1-63, XP055707940, Chalmers Universtity
- Andreasson L., Evenbratt H., Simonsson S.: "GDF5 induces TBX3 in a concentration dependent manner - on a gold nanoparticle gradient", Heliyon, vol. 6, no. 6, 10 June 2020 (2020-06-10), page e04133, XP055849634,

## Description

### Technical Field of the Invention

The invention relates to a method for differentiating stem cells into e.g. chondrocytes and integrating them into a matrix/scaffold to provide a cartilage implant. The chondrocytes, integrated into a matrix/scaffold, may be used in chondrocyte implantation. Further, the invention relates to a cartilage implant obtainable by differentiating stem cells into e.g., chondrocytes and integrating them into a matrix/scaffold.

### Background

There are several conditions, such as trauma or the disease Osteoarthritis (OA), which degenerates cartilage and/or bone structure of the patient. OA specifically is a type of joint disease that results from breakdown of joint cartilage and underlying bone. No cures exist for such cartilage and bone impairing diseases, which may lead to the need for surgical procedures to exchange the damaged tissue for a bone or cartilage implant. The human body cannot reproduce or repair cartilage on its own, since cartilage, unlike other tissues, lacks its own blood supply. Therefore, damaged cartilage has to be replaced either by implants or regenerated by the implantation of healthy cartilage cells.

Autologous chondrocyte implantation (ACI) technology has emerged over the past decade as a disease-modifying treatment with satisfying clinical results among patients suffering from isolated cartilage injuries. However, the method is complicated and very time consuming. It has therefore been considered to use stem cells of various origin to be differentiated into chondrocytes in order to not be restricted to the patient's own cells and to be able to expand a limited number of cells into chondrocytes for implantation.

Cartilage is evolved through chondrogenesis and arises from chondrocyte differentiation from mesenchymal stem cells and chondroprogenitor cells in early development. The differentiation process is initiated by mesenchymal condensation, that is, the reduction of intercellular spaces and is followed by continued differentiation. Molecules such as growth differentiation factor 5 (GDF5), transforming growth factor beta 1 (TGFβ-1), and transforming growth factor beta 3 (TGFβ-3) are known to be essential during chondrogenesis. Decreased levels of these morphogens are suggested to be involved in diseases, such as osteoarthritis (OA).

Humans are multicellular creatures, which like all multicellular organisms, are formed from one single cell, the fertilized egg. A challenge during development is how to generate distinct cell types and organs from a single cell. Organs and tissue such as cartilage and limbs are formed during development due to orchestration of growth factors that functions as morphogens, such as GDF5 and TGFβ-1 or TGFβ-3.

Various methods for controlling differentiation of stem cells have been explored, and the use of morphogenic proteins have been used in experiments in attempts to regulate e.g., the formation of tenocytes, chondrocytes and osteoblasts. Such research targets the possibility of helping patients suffering from diseases or conditions impairing their cartilage or bone formation. For instance, by deriving induced pluripotent stem cells (iPSCs) from chondrocytes, it may be possible to use an allogenic stem cell line for patients in need of treatment.

In WO 2010/051032 methods and devices for stimulating mesenchymal stem cells in a stem cell source to differentiate into osteoblasts capable of forming bone are disclosed. In WO 2013/176538, a culture medium for promoting human bone marrow mesenchymal stem cells differentiation into tenogenic lineage comprising GDF5 is disclosed, and in WO 2016/193175, a method for producing a cellular composition with *in vivo* bone and/or cartilage forming potential is disclosed. In WO 2018/174403, a stem cell loaded with nanoparticles including bone or cartilage forming agents, e.g., TGF-β (Transforming growth factor-beta), is disclosed. Further, EP3064577 provides a method for producing chondrocytes from pluripotent stem cells. The method comprising the steps of: (i) inducing pluripotent stem cells to differentiate into mesodermal cells in adherent culture, (ii) culturing the cells obtained by step (i) in adherent culture in a medium containing one or more substances selected from the group consisting of BMP2, TGFβ and GDF5, and (iii) culturing the cells obtained by step (ii) in suspension culture in a medium containing one or more substances selected from the group consisting of BMP2, TGFβ and GDF5. Furthermore, EP3446722 discloses is a three-dimensional tissue culture, comprising chondrocytes in a biocompatible artificial matrix.

However, the above mentioned prior art suffer from several disadvantages. A method displaying increased regulation and a higher reproducibility, would be preferable. Further, as reported by Li et al (Regenerative Biomaterials, 2019, 129-140, doi: 10.1093/rb/rbz022), cell lines which potentially could be used for injectable scaffolds in cartilage regeneration are limited.

Hence, there is need for an improved method for achieving differentiation of stem cells into chondrocytes, in order to be able to induce the formation of new cartilage in an efficient and reproducible manner, and to be able to provide cartilage implants.

### Summary

Consequently, the present invention seeks to mitigate, alleviate, eliminate or circumvent one or more of the above-identified potential deficiencies in the art and disadvantages singly or in any combination by providing a method for differentiating induced pluripotent stem cells (iPSCs) into chondrocytes. The chondrocytes may be integrated into a matrix/scaffold to provide a cartilage implant.

Such a method comprises the steps of seeding a surface of a substrate with iPSCs. The surface is coated with nanoparticles in a particle density of at least 500 particles/µm², and less than 1500 particles/µm², and parts of the surface in between the nanoparticles are coated with a coating agent, and growth differentiation factor 5 (GDF5) molecules are attached to the nanoparticles. The method further comprises the steps of adding a first differentiation medium to the seeded iPSCs and allowing the seeded iPSCs to differentiate at least into chondrocyte progenitor cells on the surface in the presence of the first differentiation medium.

Subsequently, the obtained cells, e.g., chondrocytes or chondrocyte progenitor cells, may integrated into a matrix/scaffold. Optionally, the obtained cells are further differentiated before being integrated into the matrix/scaffold. The obtained cells may also be further differentiated once they have been integrated into the matrix/scaffold.

In one embodiment, the method further comprises removing formed condensed chondrocyte progenitor cell aggregates from the substrate surface with GDF5 attached thereto and further differentiating the removed condensed chondrocyte progenitor cell aggregates into chondrocytes, in a second differentiation medium. This allows the condensed chondrocyte progenitor cell aggregates to differentiate into chondrocytes. The obtained chondrocytes, integrated into a matrix/scaffold, may be used in chondrocyte implantation.

In one embodiment, the first and second differentiation medium both comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof.

In a second aspect of the invention, there is provided a cartilage implant obtainable by the method for differentiating iPSCs into chondrocytes and integrating the obtained cells into a matrix/scaffold disclosed herein above.

Other objectives, features, and advantages of the present invention will appear from the following detailed disclosure, from the attached claims, as well as from the drawings. It is noted that the invention relates to all possible combinations of features.

Generally, all terms used in the claims are to be interpreted according to their ordinary meaning in the technical field, unless explicitly defined otherwise herein

### Detailed Description of Preferred Embodiments

As mentioned herein above, the human body cannot reproduce cartilage tissue when such tissue has been damaged. Hence, for most conditions causing breakdown of cartilage there is no cure available. Damaged cartilage may be replaced by implants added surgically, or cartilage can be regenerated by the implantation of healthy cartilage cells, optionally integrated into a scaffold. However, the methods presently available are time consuming and cumbersome. Furthermore, current methods for providing implantable chondrocytes (cartilage cells) often lack reproducibility and are hard to regulate, generating chondrocytes of varying quality and properties.

The present inventors envisaged that healthy chondrocytes could be produced, with high reproducibility and in a controlled way, from induced pluripotent stem cells (iPSCs) differentiating into chondrocytes in the presence of differentiation factors, *inter alia* the protein growth differentiation factor 5 (GDF5), on a substrate surface and later in a pellet formation. Such chondrocytes may be used in chondrocyte implantation.

In short, various differentiation factors were attached to nanoparticles coated on the substrate surface in a gradient pattern. This allowed for analysis of the behaviour of iPSCs seeded to said surface at different particle densities, and thereby at different concentrations of the differentiation factor.

As previously known, distinct cell types and organs in the human body are derived from a single cell. One theory on how this is achieved is that biomolecules provide signals, which induce different cellular responses. Molecules in the transforming growth factor beta (TGFβ) family are a type of signalling biomolecule, and GDF5, TGFβ-1, and TGFβ-3 are known to affect chondrogenesis (Goldring M. B. 2012). Whereas TGFβ-1 and TGFβ-3 are necessary to induce chondrogenesis from the start, GDF5 is typically considered optional.

During chondrogenesis, cartilage is generated from chondrocyte differentiation from mesenchymal stem cells and chondroprogenitor cells in early development. Chondroprogenitor cells are cells which have partly differentiated into chondrocytes. The differentiation process is initiated by mesenchymal condensation, that is, the reduction of intercellular spaces and is followed by continued differentiation.

Even though GDF5 and TGFβ molecules have previously been used in methods for differentiating stem cells into chondrocytes, there is however still a need for further methods providing increased regulation and more local and exact stimulation of the stem cells, in order to provide for differentiation with high reproducibility, in a controlled way. An issue with inducing differentiation of stem cells in various liquid medias is that controlled stimulation is hard to achieve. The stem cells in such liquid media will receive a more random stimulation from different differentiation inducing molecules depending on where they are present in the volume of the media, than if the stem cells are bound to a surface. As a result, differentiation using biomolecules such as GDF5 and TGFβ molecules to stimulate stem cells to develop into chondrocytes in a liquid medium is unpredictable and the result is hard to foresee. Further, it may be hard to direct the differentiating cells to chondrocytes rather than osteoblasts. Both chondrocytes and osteoblasts may be derived from stem cells.

Use of GDF5, TGFβ-1 and TGFβ-3 immobilized on solid surfaces to affect cells have previously been reported in a master thesis by Linnea Andreasson entitled "Effects on Chondrocyte Derived Induced Pluripotent Stem Cells Adhered to Nano Gradients Functionalized with Transforming Growth Factor Beta-I, -3 and Growth Differentiation Factor 5*".* The aim of the master thesis project was to study the cells expression of aggrecan depending on the density of proteins (TGFb-1, TGFb-3, or GDF5). This was conducted to provide another steppingstone to reveal disease mechanisms for osteoarthritis (OA), being the most common joint disease causing cartilage degeneration. Due to lack of knowledge regarding disease mechanisms, there is no existing drug based modifying therapy.

The impact of TGFβ-1, TGFβ-3 and GDF5 signalling on the expression of aggrecan in chondrocyte derived iPSCs, were elucidated by using concentration nano gradients. Whereas no specific aggrecan expression was observed for GDF5 or TGFβ-1, TGFβ-3 indicated an elevated expression at high concentrations. Further, from experiments on gradients and uniform surfaces, TGFβ-3 was indicated to be the one of the three molecules that induced aggrecan expression to the highest extent. As expected, all three molecules induced cell development.

The present inventors have elucidated a method for determining the impact of different concentrations of different biomolecules (GDF5, TGFβ-1 and TGFβ-3) on chondrocyte derived iPSCs (c-iPSCs) by using a surface with density gradient of attached gold nanoparticles to which the biomolecules were bound. Surprisingly, there was found to be preferred differentiation behavior of the seeded iPSCs at a specific particle density of GDF5-attached nanoparticles. This differentiation behavior was preferable not only in comparison to biomolecules in solution only, but also in comparison to other biomolecules attached to the nanoparticles regardless of the particle density of the gradient pattern.

In particular, GDF5 was found to induce higher levels of TBX3 (T-box transcription factor 3: considered important for, and significantly expressed during, limb formation in early development) and SOX9 (Transcription factor SOX9: chondrogenic marker expressed in chondroprogenitors and differentiated chondrocytes, being important in early cartilage development). Corresponding effects were not seen with other differentiation factors (e.g., TGFβ-1 or TGFβ-3), even though TGFβ-3 previously had been found to initially provide a higher aggrecan expression (known to be an essential feature of autologous chondrocytes, i.e. patient-derived chondrocytes) and hence represented a preferred candidate.

The effect could subsequently be reproduced with nanoparticles at a particle density within the identified range homogenously attached to the substrate surface. The local concentration of GDF5 on the substrate surface is significantly higher than the concentration that may be used in solution. Without being bound to any theory this may, at least partly, explain the result seen. Thus, not only the presence of GDF5 *per se,* but also the specific, local concentration thereof, is of importance for the effect seen. It seems that the immobilization GDF5 on a surface and the resulting dramatic increase in specificity on the molecular level (compared to methods wherein e.g., GDF5 are freely suspended in solution) provides an effect not seen with corresponding immobilization of TGFβ-1 or TGFβ-3.

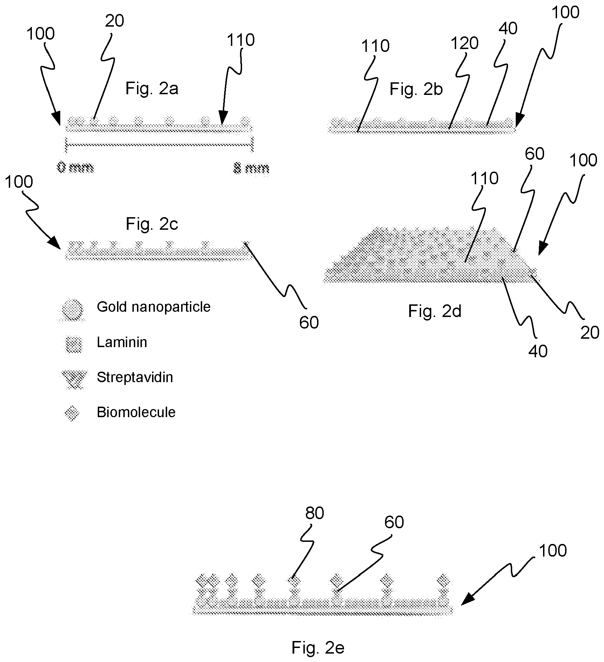

In short, to create gradient surfaces a glass substrate 100 was coated with gold nanoparticles 20 in a gradient pattern (Fig. 2a) and laminin 40 was used to cover parts 120 between the nanoparticles 20 (Fig. 2b). Streptavidin 60 was attached to the gold particles 20 (Figs 2c and 2d), and biomolecules 80 (*inter alia* GDF5) were biotinylated and attached to the streptavidin 60, whereby a surface having a gradient of biomolecules were obtained (Fig. 2e). Subsequently, c-iPSCs were seeded to the surfaces (not shown). The purpose of using gold nanoparticles was to enable binding of signalling biomolecules in a controlled and continuous gradient pattern to a stable substrate (see *"Preparation of gradient surfaces of GDF5, TGFβ-1 and TGFβ-3*" of the experimental part for more details regarding the method).

More specifically, the inventors surprisingly found that the area where nanoparticles were present at a particle density of at least 500 particles/µm² and GDF5 attached thereto gave a better differentiation result compared to other particle densities of nanoparticles (resulting in another concentration of GDF5), as GDF5 present on nanoparticles in particle density of at least 500 particles/µm² induced higher levels of TBX3 and SOX9. Further, differentiation on such a surface provided cells expressing type II collagen, one of the prominent components of cartilage.

Further, in the particle density range of 500 to 1500 particles/µm² on the substrate with GDF5 attached thereto, the seeded c-iPSCs formed so called budding zones, in which condensed cell clusters were formed. Outside this range, budding zones were not present. Since the differentiation process of iPSCs into chondrocytes is initiated by mesenchymal condensation, meaning reduction of intercellular spaces and is followed by continued differentiation, the formation of budding zones with cell clusters/aggregates may be an indication of initiation of chondrogenesis and evolvement into chondrocyte progenitor cells and eventually chondrocytes.

Though being a putative indicator of initiation of chondrogenesis, the budding zones *per se* is not sufficient for any conclusions to be drawn on differentiation of the c-iPSCs. However, as it also could be shown that the cells in the condensed cell clusters expressed elevated levels of SOX9 and TBX3, it could be concluded that GDF5 attached to particles present on a substrate in density of 500 to 1500 particles/µm² induces a preferred differentiation behaviour of c-iPSCs towards chondrocytes; especially as they also express type II collagen.

After differentiation on the substrate surface, progenitor chondrocyte cells were obtained, which could be further differentiated as a pellet suspended in a second differentiation medium, whereby chondrocytes were obtained. Various controls were used to verify the effect and the results, as will be explained more in the following.

Homogenous density surfaces (h.d. surfaces) having gold nanoparticles within the range 500 to 1500 particles/µm² and GDF5 attached thereto were subsequently produced for further analysis (disclosed in the section "*Differentiation on gradient surfaces and h.d. surfaces, and c-iPSC analysis on gradient surfaces and h.d. surfaces"* of the Experimental part). While high concentration of TGFβ-3 on gradient surfaces has been reported to increase the aggrecan expression, the gradient surfaces coated with TGFβ-1 or TGFβ-3 did still not give any satisfying results at any density along the gradient surface, as neither the expression of SOX9 nor the expression of TBX3 was significantly elevated. Still, presence of these factors is important, as recognised in the art, but there is seemingly no need or advantage of coating them on a surface. Further, cells differentiated on surfaces coated with TGFβ-3 did express nearly exclusively type I collagen, and only very little type II collagen.

Hence, an embodiment of the invention relates to a method for differentiating induced pluripotent stem cells (iPSCs) into chondrocyte cells and integrating them into a matrix/scaffold to provide a cartilage implant. The differentiation takes place in a stable and controllable environment, and provides a continuously foreseeable result. The cells to be integrated into the matrix/scaffold may be chondrocyte cells, or they may be chondrocyte progenitor cells, to be further differentiated into chondrocyte cells within the matrix/scaffold. Further, another embodiment of the invention relates to the thereby provided chondrocytes, i.e. by differentiating induced pluripotent stem cells (iPSCs) into chondrocyte cells, for use in chondrocyte implantation. The chondrocytes for use in chondrocyte implantation are integrated into a matrix/scaffold. Correspondingly, the thereby provided chondrocytes may be used in a chondrocyte implantation method, i.e. the provided chondrocytes may be implanted into a subject in need thereof.

The method will be explained more detailed in the following, with reference to the Figures 2a-2e and comprises seeding a surface 110 of a substrate 100 with iPSCs, when the surface 110 is coated with nanoparticles 20 having a particle density of at least 500 particles/µm². The parts 120 of the surface 110 in between the nanoparticles 20 are coated with a coating agent 40, and GDF5 molecules are attached to the nanoparticles 20. A first differentiation medium is added to the seeded iPSCs and the seeded iPSCs are then allowed to differentiate at least into chondrocyte progenitor cells on the surface 110 in the presence of the first differentiation medium.

Hence, the method may comprise using either nanoparticles coating the glass substrate surface 110 in a gradient pattern, or having nanoparticles coating the surface 110 homogenously. The nanoparticle gradient surface results in a gradient pattern of GDF5 molecules attached thereto, and a homogenous density of nanoparticles results in that GDF5 has a homogenous density when attached thereto. According to an embodiment, the nanoparticles coat the glass substrate surface 110 homogenously.

Chondrocyte progenitor cells are cells which have differentiated from iPSCs into a pre-stage of chondrocyte cells. They have a constrained differentiation potential and a lower capacity for self-renewal, indicating a more mature stage than iPSCs, and are a key stage in the differentiation into chondrocytes.

The coating agent 40 may be a proliferative agent, such as laminin used in the experiments disclosed herein. However, other proliferative agents, such as extra cellular matrix proteins (ECM proteins), e.g. laminins and fibronectins, and cell specific cytokines, or a combination thereof may be used for the method disclosed herein. The laminin used herein does not only cover and thereby block the parts 120 between the gold nanoparticles 20, such that unwanted attachment of the biomolecules to the surface 110 is prevented; the laminin also, at least to some extent, stimulates cell differentiation.

Further, it has been shown that the c-iPSCs have an impaired capacity to attach to glass surfaces. Laminin therefore also served as an agent which successfully improved cell adhesion to the substrate.

Hence, coating agents in general have several purposes, e.g., enhancing binding properties of cells to glass surfaces and preventing unwanted adhesion of biomolecules to substrate surface instead of binding to wanted particles (in this case, the streptavidin coated gold nanoparticles).

The iPSCs may be differentiated on the substrate surface 110, for between 3 and 10 days, preferably between 4 and 8 days. The upper time limit of 10 days for the differentiation on the substrate surface is limited by the adherence of the iPSCs to the substrate 100. After 10 days, it is hard to maintain the iPSCs on the substrate surface 110. As such, the differentiation on the substrate surface may continue as long as the cells are able to be maintained at the substrate. The lower limit of 3 days is the preferred minimum time required for the differentiation on the substrate surface 110. Less than 3 days will probably not result in sufficient stimulation by GDF5 and adequate differentiation occurring.

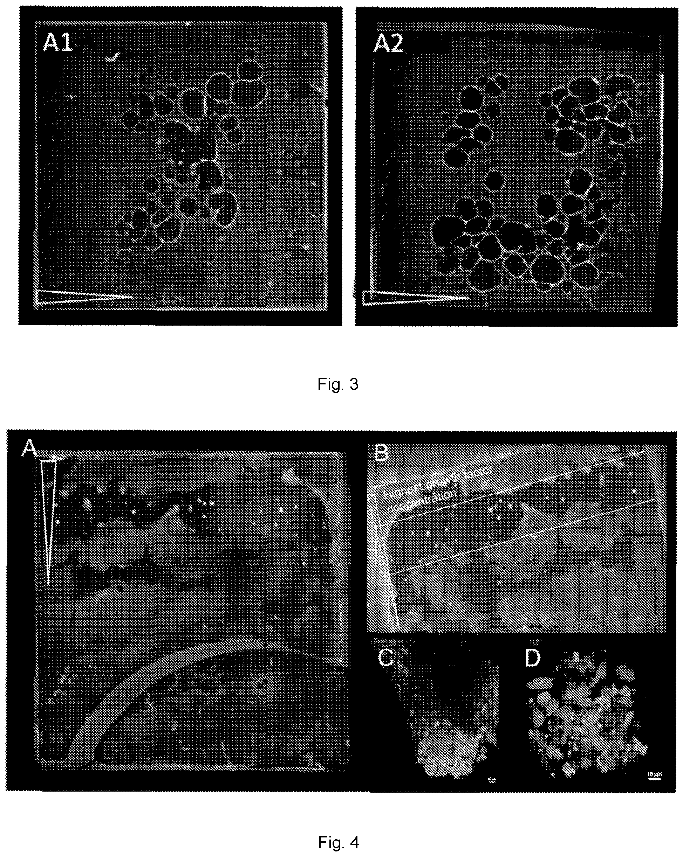

After a few days, such as 5 days as applied in the experimental example disclosed in the result section of the part "*Differentiation on gradient surfaces and h.d. surfaces, and c-iPSC analysis on gradient surfaces and h.d. surfaces* ", the cells will form cell aggregates, also referred to as clusters herein, in budding zones on the substrates. The term budding zone describes the appearance of the surfaces and a zone in which cell clusters form cell buds or nodular structures, which are separated from a larger crowd of cells. For instance, in Fig. 4 in section B, a light grey area forms a carpet like structure of cells. The white dot-like formations are condensed cell clusters (nodular structures/nodules) and at the end of the light grey area within the white lines, a budding zone is shown. At a top of a triangular shaped light grey area within the white lines, a white separate nodule/bud is about to bud off within the budding zone. The budding zones are of interest since the formation of the cell aggregates in the budding zones indicate an initiation of chondrogenic behaviour.

An embodiment thus relates to said method which further comprises removing formed condensed chondrocyte progenitor cell aggregates from the substrate surface 110 having GDF5 attached thereto. Once removed, the condensed chondrocyte progenitor cell aggregates are further differentiated in a second differentiation medium. In further differentiating the progenitor cells, they may be present within a matrix/scaffold to be integrated therein. Alternatively, the cells are integrated into the matrix/scaffold once they have been further differentiated in the second differentiation medium. According to such an embodiment, comprising further differentiating progenitor cell aggregates in a second differentiation medium, the further differentiation is preferably performed for 4 to 10 weeks, such as for 5 to 8 weeks. Further differentiation for less than 4 weeks has low likelihood of resulting in obtained chondrocytes.

Preferably, the removed condensed cell aggregates are formed into a three-dimensional pellet structure, before being further differentiated. The removed condensed cell aggregates were combined into a pellet structure since further condensation increases a cartilage-like structure and induces secretion of cartilage-specific matrix. The removal of the condensed chondrocyte progenitor cell aggregates may be conducted by releasing the cells from the gradient surface or h.d. surface and a separation of the condensed chondrocyte progenitor cell aggregates, followed by centrifuging the condensed chondrocyte progenitor cell aggregates, such that the pellet structure is obtained. The cells may be released by adding trypsin. Once released, the action of trypsin may be quenched by adding human serum. Details regarding obtaining such pellets and further differentiation is disclosed in *"Pellet cultures from GDF5 gradient surfaces, GDF5 h.d. surface and chondrocyte control experiments* ".

The first and second differentiation medium may be of the same kind. An exemplary composition of the differentiation medium is disclosed in Table 1 herein below. The first and/or the second differentiation medium, may according to an embodiment comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof. Other differentiation medium known in the art may also be used. The differentiation medium provides additional factors, *inter alia* TGFβ-1 and TGFβ-3, of importance for cell growth and differentiation. The differentiation medium may comprise 2 to 30 ng/mL, such as 5 to 15 ng/mL, preferably 10 ng/mL of TGFβ-1 and/or 2 to 30 ng/mL, such as 5 to 15 ng/mL, preferably 10 ng/mL of TGFβ-3.

According to an embodiment, a maintenance medium is added to the substrate surface 110 after the iPSCs have been seeded to the surface 110. The maintenance medium is preferably added before the differentiation medium is added. The maintenance medium may for instance be *Cellartis*^{®} *DEF-CS*^{™} *500 Basal Medium with Additives.* However, any other known maintenance medium known in the art may be used. The purpose of the maintenance medium is to maintain the viability and physiological characteristics of the cell culture until they have attached to the surface and are ready to start the differentiation process.

The iPSCs used herein may be c-iPSCs (chondrocytes derived iPSCs) generated by reprogramming chondrocytes using a preferably footprint-free method based on mRNA delivery. Stem cells are cells that can differentiate into other types of cells and can also divide in self-renewal to produce more of the same type of stem cells. Mammals comprise two types of stem cells, i.e. embryotic stem cells and adult stem cells, the latter also referred to as somatic stem cells. Somatic stem cells are derived from tissue samples from (healthy) adults. iPSCs are pluripotent stem cells, meaning they may differentiate into different types of cells, thus being a promising starting point for regenerative medicine. Since iPSCs may be derived from somatic stem cells, there is no need for the use of embryotic stem cells, though embryotic stem cells may be used. According to an embodiment, the iPSCs are not derived from embryotic stem cells. As known in the art, iPSCs may be formed by exposing cells, such as chondrocytes, to reprogramming factors, which reprograms the cells into iPSCs.

The culturing of cells disclosed herein is explained in the section *"Cell culturing*" of the Experimental part. An advantage in using chondrocytes and reprogramming them to c-iPSCs is that iPSCs have been shown to more easily differentiate along lineages related to the cell type of origin, probably due to a residual epigenetic memory (Borestrom, C., S. Simonsson, et al 2014). In addition, the ethical issues concerning human embryonic stem cells (hESCs) are avoided since chondrocytes for reprogramming can be obtained by healthy individuals.

In one embodiment, 20 000 to 100 000 iPSCs/cm², preferably 30 000 to 70 000 iPSCs/cm², most preferred 50 000 cells/cm², are seeded to the substrate surface 110.

In the experiments disclosed herein, the nanoparticles 20 are gold particles coated with thiolated streptavidin, and the GDF5 molecules are biotinylated and attached to the nanoparticles through biotin/streptavidin interaction. However, any such known principle for facilitating binding and interaction between nanoparticles and a biomolecule may be used, since the biotin/streptavidin interaction is only an interaction according to one embodiment. For instance, such interaction may be formed using an ion-exchange/interaction, hydrophobic interactions and dative binding, covalent binding of thiols, carbo di-imide chemistry, bi-functional linkers (such as EDC/NHS chemistry) or click chemistry.

Alternatively, the molecules may be purchased already provided with a biotin or another type of interaction marker. The method used for the biotinylation is disclosed in the section of the Experimental part. In addition, other methods for biotinylation known in the art may be used.

In tissue engineering, scaffolds are designed to provide an environment for the formation and integration of viable tissue. Scaffolds tend to mimic an extracellular matrix of a certain tissue and are engineered to provide cellular interactions stimulating cell growth. Tissue scaffolds can be formed of different materials, for instance natural or synthetic materials, several used materials are biodegradable.

The method may thus further comprise integrating the obtained differentiated cells, e.g., chondrocytes or chondrocyte progenitor cells, into a matrix and/or scaffold, such as in an injectable scaffold. Integration of differentiated cells into a matrix and/or scaffold, e.g., a hydrogel, can be realized by either seeding chondrocytes into a prefabricated porous scaffold, or by encapsulating cells during scaffold formation. Such integration may cause the chondrocytes (or chondrocyte progenitor cells) to further divide and reproduce themselves and form a biological implant, which may replace damaged cartilage in a subject. Hence, the present disclosure also concerns a cartilage implant, made of a scaffold and/or matrix comprising chondrocytes which have been obtained by differentiating iPSCs into chondrocytes by the method disclosed herein.

There are reports in the art (cf. Biopolymer-Based Hydrogels for Cartilage Tissue Engineering, by Biji Balakrishnan and R. Banerjee, Chemical reviews, 2011, p. 4453-4474, dx.doi.org/10.1021/cr100123h; and Advances of injectable hydrogel-based scaffolds for cartilage regeneration, by Jiawei Li, Guojun Chen, Xingquan Xu, Peter Abdou, Qing Jiang, Dongquan Shi, and Zhen Gu, Regenerative Biomaterials, 2019, p. 129-140, doi: 10.1093/rb/rbz022) concerning the use of hydrogels as matrixes and/or scaffolds for cartilage implants, such as articular cartilage implants. As can be recognised, hydrogels represent a preferred type of matrix and/or scaffold for integrating chondrocytes into. The hydrogel to be used as a, or as part of a, matrix and/or scaffold may be based on a biopolymer, such as a polysaccharide (e.g., hyaluronate, chondroitin sulfate, chitosan, alginate, and agarose) or a protein (e.g., silk fibroin, fibrin, gelatine, and collagen). Further, among synthetic polymers, poly ethylene glycol (PEG) has been extensively investigated for biomedical applications because of its good tissue compatibility, nontoxicity and hydrophilicity, and does hence represents a preferred synthetic polymer.

Further, the implant obtained by differentiating iPSCs into chondrocytes and integrating them into a matrix/scaffold is useful as cartilage implant. Hence, an embodiment relates to a cartilage implant obtainable by differentiating iPSCs into chondrocytes and integrating the obtained chondrocytes into a matrix/scaffold, e.g., a hydrogel, in accordance with the method disclosed herein.

Furthermore, the chondrocytes obtained by differentiating iPSCs into chondrocytes are useful in chondrocyte implantation. Hence, an embodiment relates to a chondrocyte obtained by differentiating iPSCs into chondrocytes by the method disclosed herein for use in chondrocyte implantation. Correspondingly, the chondrocytes obtained by differentiating iPSCs into chondrocytes may be used in a chondrocyte implantation method, i.e. the chondrocytes may be implanted into a subject in need thereof. Thus, the chondrocytes may be provided by the present method and subsequently implanted into a subject in need thereof, such as into the knee of a subject suffering from osteoarthritis of the knee.

Without further elaboration, it is believed that one skilled in the art can, using the preceding description and the following experimental part, utilize the present invention to its fullest extent. The preferred specific embodiments described herein are, therefore, to be construed as merely illustrative and not limitative of the remainder of the description in any way whatsoever. Further, although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g., different than those described above.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous.

In addition, singular references do not exclude a plurality. The terms "a", "an", "first", "second" etc. do not preclude a plurality.

### Brief description of the Drawings

By way of example, embodiments of the present invention will now be described with reference to the accompanying drawings, in which:
**Fig. 1a** shows a montage of SEM pictures taken at different positions with 1 mm interval along a gold nanoparticle gradient surface, the scale bar indicates 200 µm. The gradient is verified up to 8 mm on a 18x18 mm silica surface;
**Fig. 1b** shows a diagram of the gradient profile for a gold nanoparticle gradient functionalized with GDF5. The x-axis indicates gradient distance in mm and the y-axis shows GDF5 particles/µm² as a function of the gradient distance;
**Fig. 2a** shows a schematic glass surface being 8 mm wide and provided with gold nanoparticles attached in a gradient pattern on a glass substrate;
**Fig. 2b** shows the schematic glass surface of Fig. 2a, having Laminin molecules attached to the glass surface between the gold nanoparticles, to avoid unwanted adherence of other molecules to the glass surface between each gold nanoparticle and aid the cell adherence to the surface and the differentiation of the cells;
**Fig. 2c** shows the schematic glass surface of Fig. 2b having Streptavidin attached to the gold nanoparticles. The Streptavidin will act as a linker to biotinylated biomolecules;
**Fig. 2d** shows a perspective view of the schematic glass surface of Fig. 2c. The gradient of Streptavidin coated gold nanoparticles is ready to be functionalized by biotinylated morphogens;
**Fig. 2e** shows a functionalised glass substrate surface, where biotinylated biomolecules have been attached to the Streptavidin. In the present disclosure, said biomolecules are GDF5, TGFβ-1 and TGFβ-3;
**Fig. 3** shows laminin control gradient surfaces (A1, A2). The gradient surfaces were visualized with IN CELL Analyzer 6000 (IN Cell 6000, GE Healthcare, United Kingdom). Cavities with no cells in the centre were formed, mainly at higher laminin densities. The bars on each surface A1, A2 indicate the direction of the nanoparticle density gradient with laminin coupled to the surface;
**Fig. 4** shows the budding zone in (A) a GDF5 gradient surface, 18x18 mm, seeded with c-iPSCs visualized using high throughput confocal IN CELL Analyzer 6000. The bar on the left upper side indicates the extent of the gradient where the continuous density increase is shown with a marker on the left-hand side, with low GDF5 density at the bottom of the image and high GDF5 density at the top, (B) focusing on the budding cell clusters identified at particle density of 500-1500 gold nanoparticles/µm². Two white lines indicate the budding zone, and (C-D) show close up pictures of buds identified in the budding zone shown in (B), the scale bars in (C) and (D) are 10 µm;
Fig. 5A-F all show comparative pictures on cell behavior on GDF5, TGFβ-1 and TGFβ-3 h.d. surfaces, 18x18 mm, visualized using high throughput confocal IN CELL Analyzer 6000;

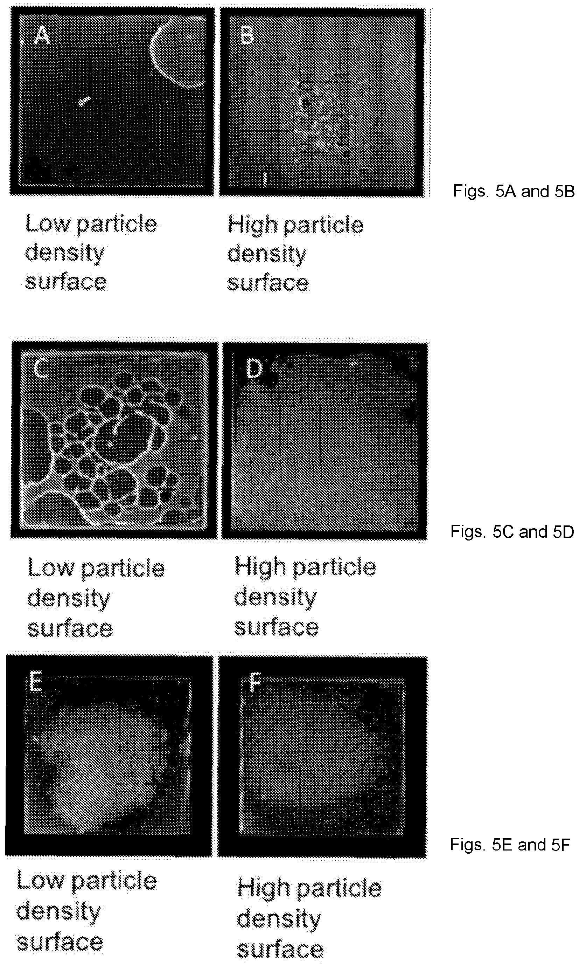

**Fig. 5A** shows GDF5 surfaces with a low particle density at 400 particles/µm². No budding zones are identified;
**Fig. 5B** shows a GDF5 surface with a particle density in the range of the budding zone at 900 particles/µm². Buds are clearly identified as brighter colored clusters;
**Fig. 5C** shows TGFβ-1 surfaces with a low particle density at 600 particles/µm². Cell-free cavities were formed, a response comparable to cells on laminin only (Fig. 3);
**Fig. 5D** shows evenly distributed cells on TGFβ-1 surfaces with a high particle density of 2000 particles/µm²;
**Fig. 5E** shows TGFβ-3 surfaces with low density, 600 particles/µm², and;
**Fig. 5F** shows TGFβ-3 surfaces with high density, 1900 particles/µm²;

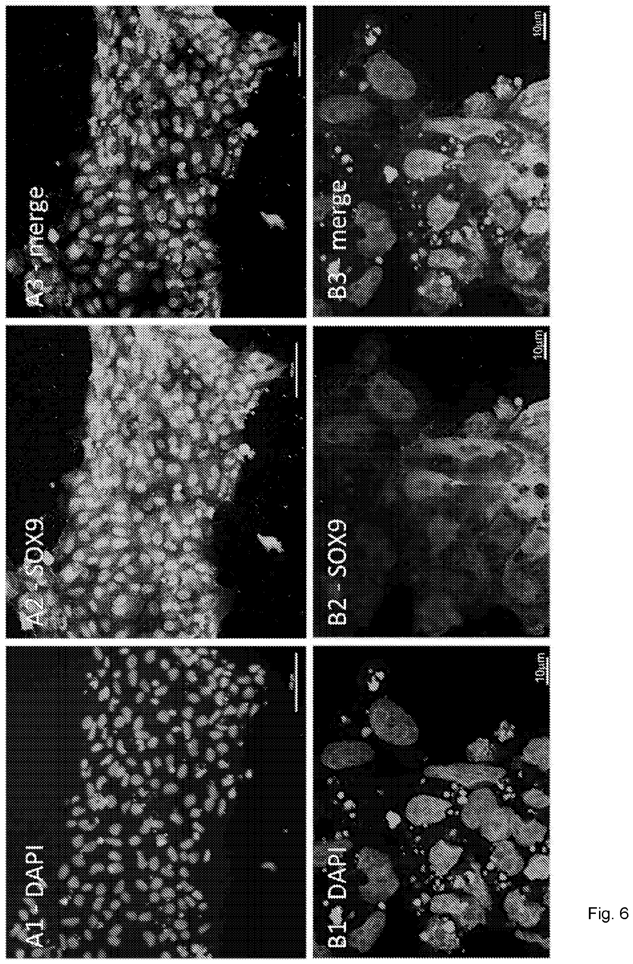

**Fig. 6** shows immunostaining and a marked expression of SOX9 in c-iPSCs after five days of differentiation on a GDF5 gradient. (A1-A3) Images were acquired in the budding zone using florescence microscopy, 20X objective. Scale bars denote 100 µm. (B1-B3) Images were acquired in the budding zone with a confocal microscope, 40X objective. Scale bars denote 10 µm;

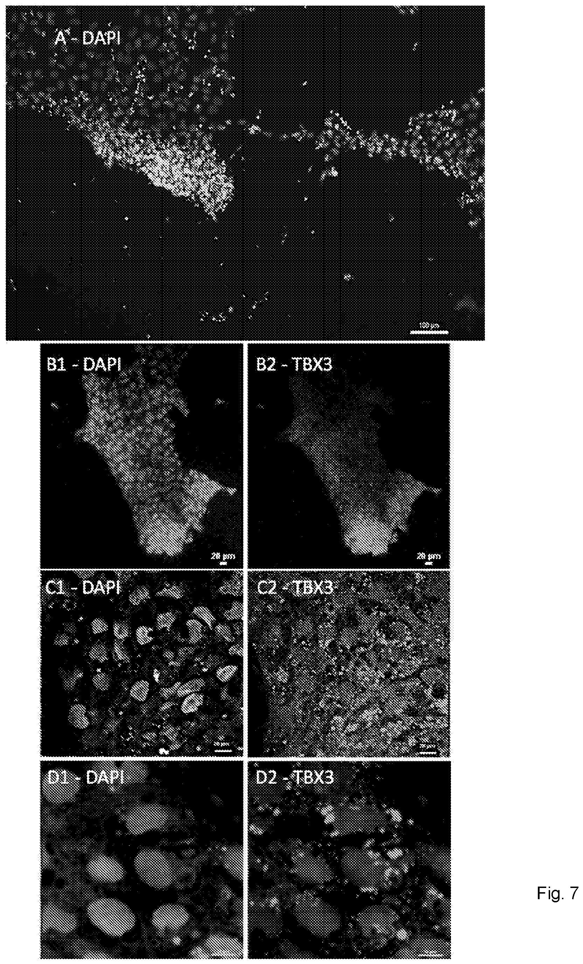

**Fig. 7** shows that TBX3 are localized in the nuclei and in vesicles in the budding zone. TBX3 expression is visualized after five days of differentiation on a GDF5 gradient. (A) shows a budding zone on a GDF5 gradient. Scale bar 100 µm. (B1-B3) and (C1-C3) show two different budding clusters. Images were acquired with a confocal microscope, 40X objective. Scale bars 20 µm. (D1-D3) is a closeup of a budding cluster. Images were acquired using a 60X objective. Scale bars 10 µm.

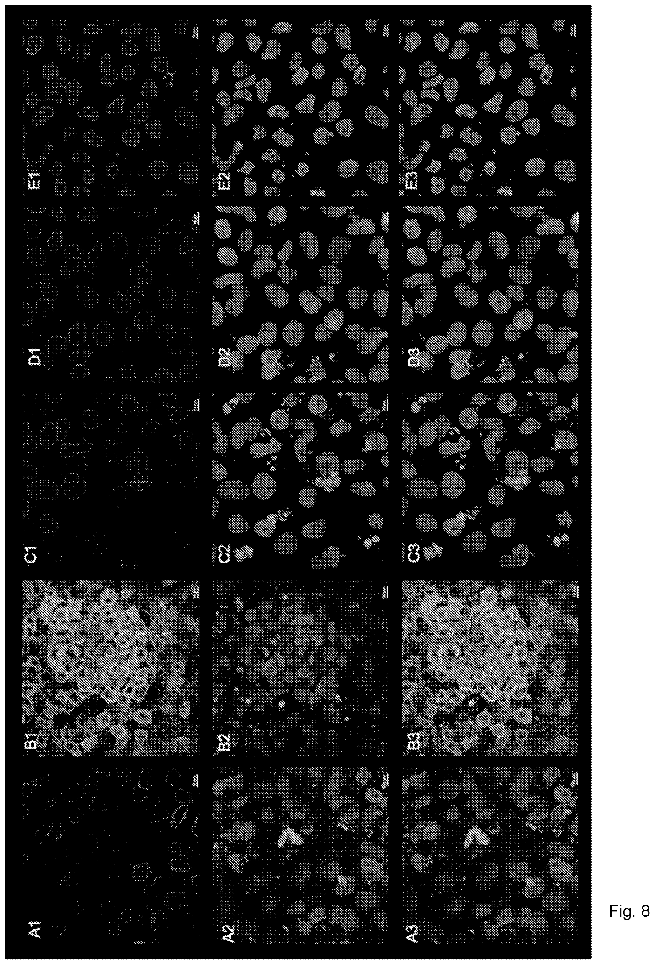

**Fig. 8** shows iPSCs on h.d. surfaces immunostained with TBX3 monoclonal (Table 2) antibody, using confocal microscopy. TBX3 expression is clearly upregulated on the GDF5 h.d. surface with a particle density in the range of the budding zone, compared to all other surfaces. (A) shows a GDF5 surface with low particle density. (B) is a GDF5 budding zone surface. (C) shows a TGFβ-3 surface with the same particle density as (B). (D) is a TGFβ-3 surface with the same particle density as the picture (B) but with GDF5 (10 ng/ml) added in the differentiation medium. (E) is a surface without biomolecules, coated with Coat-1 (DEF-CS 500 Coat-1, Cellartis, Sweden); and

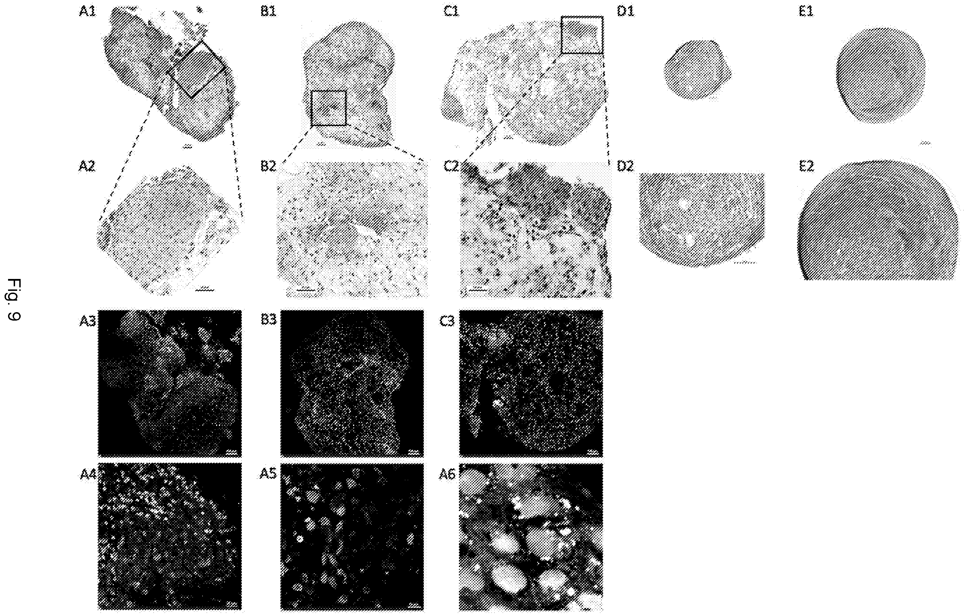

**Fig. 9** shows histological sections of pellets generated from c-iPSCs (A-C) and, as a control, from patient-derived chondrocytes (D-E). A1, A2 and E1, E2 were stained with Alcian Blue van Gieson that demonstrates the presence of proteoglycans and collagens. A3-A6 & B3-C3 were immunostained for TBX3 which were especially upregulated in the areas with higher amounts of GAG. (A1-A6) show c-iPSCs differentiated on a GDF5 h.d. surface with low particle density. (B1-B3) show c-iPSCs differentiated on GDF5 h.d. surface with budding zone particle density. (C1-C3) are c-iPSCs differentiated on GDF5 gradient surface. (D1-D2) show chondrocyte pellet differentiated on GDF5 gradient surface. (E1-E2) show a chondrocyte pellet with 5% human serum in the medium. In A1-E1, A3-C3, A5-A6, the scale bars indicate 100 µm. In A2-E2, the scale bars indicate 50 µm. In A4, the scale bar indicates 20 µm.

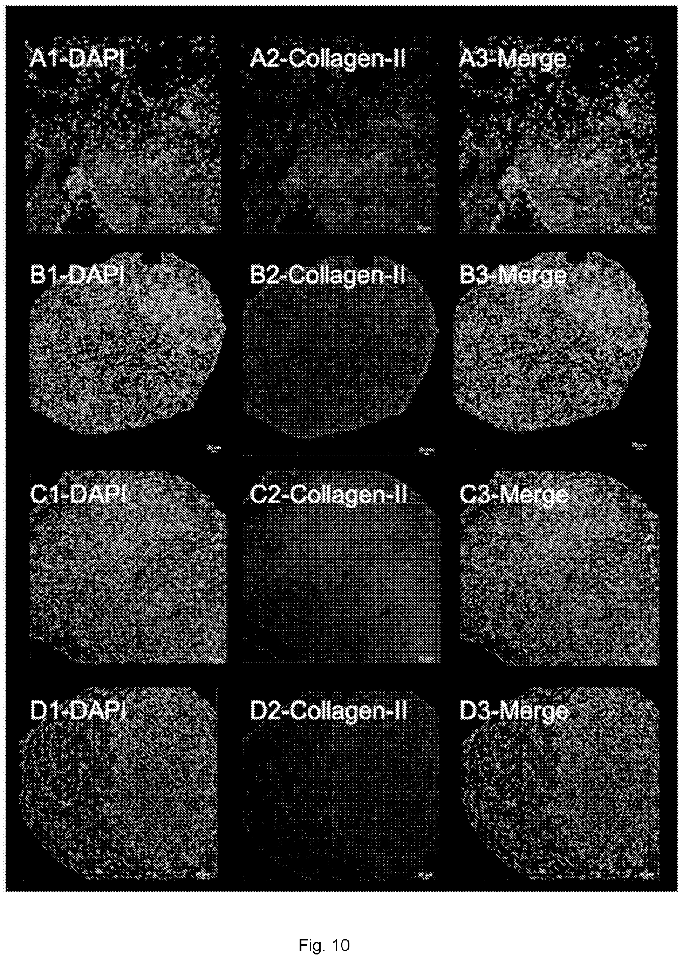

**Fig. 10** shows histological stained sections of pellets generated from c-iPSCs (A) and patient-derived chondrocytes (B-D). It shows the collagen II expression in chondrocytes derived from c-iPSCs first differentiated on a GDF5 gradient (A2) in comparison to the collagen II expression in patient-derived chondrocytes maintained in pellet with 5% serum for two weeks (B2), in patient-derived chondrocytes maintained five days on GDF5 gradient, followed two weeks in pellet in differentiation medium (C2), and in patient-derived chondrocytes maintained five days on TGFβ-3 gradient, followed two weeks in pellet in differentiation medium (D3). A1, B 1, C1, and D1 show DAPI stained cell cores. A3, B3, C3, and D3 show the merged channels. Scale bars 20 µm.

### Experimental

The following examples are mere examples and should by no means be interpreted to limit the scope of the invention, as the invention is limited only by the accompanying claims.

### Abbreviations

- OA: Osteoarthritis
- ACI: autologous chondrocyte implantation
- c-iPSC: chondrocyte derived induced pluripotent stem cells
- iPSC: induced pluripotent stem cells
- TGFβ-1: transforming growth factor beta 1
- TGFβ-3: transforming growth factor beta 3
- GDF5: growth differentiation factor 5
- BMP14: bone morphogenetic protein 14
- BMPs: bone morphogenetic proteins
- ECM: extracellular matrix
- TBX3: T-box transcription factor 3
- DAPI: 4',6-diamidino-2-phenylindole
- PBS: phosphate buffered saline
- GAGs: glycosaminoglycans
- SOX9: Transcription factor SOX9
- PDGF: platelet-derived growth factor
- h.d. surface: homogenous density surface

### Cell culturing

### Method - generating c-iPSCs from chondrocytes

It has previously been shown that chondrocytes from autologous chondrocyte implantation (ACI)-donors can be reprogrammed into iPSCs using a footprint-free method based on mRNA delivery (Borestrom, C., S. Simonsson, et al 2014), a method that was applied to generate c-iPSCs for this study. The iPSCs were derived from chondrocytes from an anonymous female donor.

In short, the method involves obtaining chondrocytes from ACI-donors, isolating said chondrocytes and expanding them in a chondrocyte medium. Non-integrating mRNA reprogramming technology was used, and mRNA reprogramming was conducted using the Stemgent mRNA Reprogramming Kit according to the manufacturer's instructions, with some minor modifications, performing daily mRNA transfections for 21 days. Clonal iPSC lines were established by picking hESC-like colonies.

### Method- cell culturing of c-iPSCs for control experiments

The iPSCs were stored in liquid nitrogen at -196°C until use. Newly thawed c-iPSCs were seeded for monolayer culturing in cell culture flasks (Corning^{™} Primaria^{™} Tissue Culture Flasks, vented, FisherScientific, USA) which were coated specifically for c-iPSCs (DEF-CS 500 Coat-1, Cellartis, Sweden) and placed in a 37°C humidity chamber at 90% humidity and 5% CO₂. Cell medium (Cellartis^{®} DEF-CS^{™} 500 Basal Medium with Additives, Cellartis, Sweden), supplemented with additional growth factors, was changed every day and cell passage was performed every third day. All handling of cells was performed in a sterile cell lab area.

### Method- cell culturing of chondrocytes for control experiments

The chondrocytes used for the pellet-experiments were provided from three combined anonymous male donors, with written consent. Chondrocytes were expanded in a monolayer in chondrocyte medium consisting of Dulbecco's modified Eagle's medium/F12 (DMEM/F 12, ThermoFisher Scientific, USA) supplemented with 0.5 mL 8 mM L-ascorbic acid (Sigma Aldrich, USA), 1% penicillin-streptomycin (Sigma Aldrich, USA) and 10% human serum, at 37°C in 5% CO₂ and 90% relative humidity. Medium was changed three times per week. All handling of cells was performed in a sterile cell lab area.

### Preparation of gradient surfaces of GDF5, TGFβ-1 and TGFβ-3

### Method

Cline Scientific's Nano Gradients and Nano Surfaces (Cline Scientific AB, Sweden) were used as a tool in the differentiation process. The gradient surface may be a nano gradient surface as disclosed in EP 2 608 896 B1. Such nano gradient surface has a continuous gradient of deposited and electrically charged nanoparticles. The nanoparticles preferably have an average diameter between 10 and 60 nm, and the average centre-to-centre distance of the nanoparticles is typically about 10 to 60 nm in one end of the gradient and about 100 to 150 nm in the other end of the gradient.

A particle density of nanoparticles on the gradients were in the range of from 3 to 3000 particles/µm₂, determined with Scanning Electron Microscopy (SEM) using a Zeiss Ultra 55 operating at an accelerating voltage of 3.00 kV. Images were acquired in the secondary/backscattered electron mode using the In Lens detector. The nanoparticles used were gold nanoparticles and the surface was a silica surface. The substrate 100 is shown schematically in **Figs. 2a-2e****.**

According to a functionalization protocol, thiolated streptavidin 60 (SH-streptavidin, ProteinMods, USA) was applied to the gold nanoparticles 20, as shown in **Fig. 2c****.** After sufficient incubation in room temperature, superfluous streptavidin 60 was rinsed off with Phosphate Buffered Saline (PBS) (Phosphate Buffered Saline, Amresco, USA).

Thereafter, laminin 521 (Biolaminin 521 LN, BioLamina, Sweden) was applied to coat parts 120 of the glass surfaces 110 between the nanoparticles. After incubation (as above), superfluous laminin 40 was rinsed off with PBS. The substrate 100 and its prepared surface 110 having a gradient of gold nanoparticles 20, having thiolated streptavidin 60 attached to said nanoparticles 20, and where the surfaces 110 in between the nanoparticles 20 are coated with laminin 40 is shown in **Fig. 2c** and **2d****.**

Previous experiments with glass surfaces showed that the c-iPSCs have an impaired capacity to attach to glass. Therefore, laminin was attached to the gradient surface between the biomolecule functionalized particles, successfully ameliorating cell adhesion (Aumailley, M. 2013). In addition, laminin will overall enhance the binding of iPSCs to the surface and is thought to stimulate cell differentiation.

GDF5 (R&D Systems, Bio-Techne, MN, USA), TGFβ-1 (R&D Systems, Bio-Techne, MN, USA) and TGFβ-3 (R&D Systems, Bio-Techne, MN, USA) were biotinylated to facilitate binding to streptavidin. The biotinylation was performed with a superfluous amount of biotin (EZ-LinkTM Sulfo-NHS-LC-Biotin, Thermo Fischer Scientific, USA) to make sure that biotin was attached to all available sites of the proteins GDF5, TGFβ-1 and TGFβ-3.

The excess amount of biotin that was not attached was discarded using repeated wash and centrifugation according to Thermo Fisher's biotinylation protocol. The success of the biotinylation was controlled using a HABA test (HABA/Avidin Reagent, Sigma Aldrich, USA). The HABA test ensured that the number of biotins attached to the proteins were within specified limits according to the producers.

The biotinylated proteins 80 with a known concentration, 40nM in suspension, were attached to the streptavidin 60 coated gold nanoparticles 20 and incubated at 4°C overnight. Similarly, to the previous steps, superfluous proteins (GDF5, TGFβ-1 and TGFβ-3) were rinsed off with PBS. After functionalisation the surfaces were stored in 4°C until c-iPSCs were seeded and differentiated on the gradient surfaces. A functionalised gradient surface 100 is shown schematically in **Fig. 2e****.**

During SEM analysis of the gradient surfaces, the particle density where the c-iPSCs had a response that was of interest were identified (500 to 1500 particles/µm²). Densities within the range of interest were reproduced on new homogenous density (h.d.) surfaces (without a gradient pattern) (Nano Surfaces, Cline Scientific AB, Sweden).

H.d. surfaces with a particle density lower (400 particles/µm²) than the particle density of interest (500 to 1500 particles/µm²) were used as controls.

Gradient surfaces with only streptavidin and laminin were used as reference surfaces.

Several h.d. surfaces within the range of 500 to 1500 particles/µm² were reproduced and tested, among others 575 particles/µm², 652 particles/µm², 729 particles/µm², 768 particles/µm², 792 particles/µm², 859 particles/µm², 1017 particles/µm², 1027 particles/µm², 1083 particles/µm², 1145 particles/µm², and 1367 particles/µm².

The h.d. surfaces and control surfaces were prepared using the same method as described above.

### Results

Throughout the experiments, the gradient surfaces were necessary material to study how the cells are affected by morphogen protein at different concentrations.

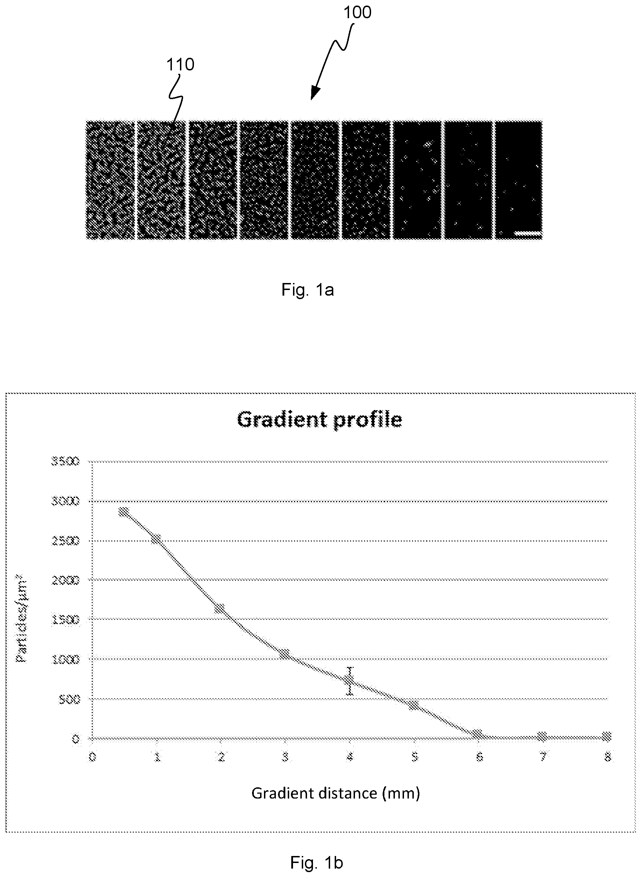

A montage of SEM pictures taken at different positions with 1 mm intervals along the substrate 100 having a gradient surface 110 is provided in Fig 1. A schematic view of such substrate 100 with the gradient surface 110 is provided in **Fig. 2a****.**

**Fig. 2e** shows a schematic illustration of how the morphogens 80 are attached to the gold nanoparticles 20 in a gradient pattern via the linker streptavidin 60 and with laminin 40 between particles to ameliorate cell adhesion.

As described above, after functionalization of the gradients, seeding and differentiation of c-iPSCs, the gradient surfaces were scanned to visualize the cells. New h.d. surfaces having particle densities in the range where c-iPSCs had shown favourable response were produced, based on the cell response found on the gradient surfaces.

The resulting cell behaviour from gradient surfaces and h.d. surfaces functionalized with morphogen proteins and laminin are presented below.

### Differentiation on gradient surfaces and h.d. surfaces, and c-iPSC analysis on gradient surfaces and h.d. surfaces

### Method - differentiation on surfaces

Each gradient surface and h.d. surface were seeded with 50 000 cells/cm² and incubated in 6 well plates at 37 °C in a humidity chamber for two hours. Thereafter, 2 ml maintenance medium was applied to each well. The maintenance medium was a commercially available medium (Cellartis^{®} DEF-CS^{™} 500 Basal Medium with Additives, Cellartis, Sweden).

The next day, 2 ml chondrocyte differentiation medium, a previously published modified medium (Nguyen, Hagg et al. 2017) (Table 1), was added with different compositions depending on which morphogen the gradient surface was functionalized with.

To gradient surfaces functionalized with TGFβ-1, TGFβ-3 was added in the differentiation medium.

To gradient surfaces functionalized with TGFβ-3, TGFβ-1 was added to the differentiation medium.

GDF5 gradient surfaces had both TGFβ-1 and TGFβ-3 added in the differentiation medium, as they are known to be essential for differentiation of c-iPSCs into chondrocytes.

The differentiation medium was changed every second day during the five-day differentiation period.

### Method - c-iPSC analysis

In order to analyse a middle step of the differentiation process, the results from the differentiation on the surfaces (both controls, gradient surfaces and h.d. surfaces) were analysed. Hence, this method step is not necessary to differentiate the c-iPSCs to chondrocytes but serves to provide insight into the differentiation process in the first place.

Previous preliminary studies performed by the inventors using c-iPSCs and patient-derived chondrocytes for differentiation on gradient surfaces showed that especially chondrocytes were difficult to maintain attached to the gradient surface after five days. Therefore, after five days of differentiation on the gradient surfaces in differentiation medium, the cells were fixed with HistofixTM (Histolab Products AB, Sweden), nuclei were stained with 4',6-diamidino-2-phenylindole (DAPI) (ProLongTM Gold Antifade Mountant with DAPI, Thermo Fisher Scientific, USA), and the surfaces were mounted on glass slides. Cell analysis was performed using the fluorescence imaging microscope IN Cell Analyzer 6000 (IN Cell Analyzer 6000, GE Healthcare, United Kingdom), Eclipse 90i (Eclipse 90i, Nikon Instruments, Japan) and Confocal microscope (Nikon A1, Nikon Instruments, Japan).

The results from the cell analysis for the different surfaces produced are presented below.

**Table 1 - Differentiation medium¹**

| **Component** | **Concentration** | **Company** |
|---|---|---|
| DMEM, high glucose, L-Glutamine and HEPES. PEST added | 4500 mg/L | Gibco |
| Insulin-Transferrin-Selenium | 1/0.55/0.00067 g/L | Thermo Fisher Scientific |
| Ascorbic acid | 14 µg/ml | Sigma Aldrich |
| Dexamethasone | 0.1 µM | Merck |
| Linoleic acid | 5 µg/ml | Sigma Aldrich |
| Sodium Pyruvate | 1 mM | Sigma Aldrich |
| TGFβ-1 | 10 ng/ml | R&D Systems |
| TGFβ-3 | 10 ng/ml | R&D Systems |

| | | |
|---|---|---|
| *Components and concentrations of the used for differentiation of c-iPSCs and for pellet culture. The first six components were always included in the medium. TGFβ-1 and TGFβ-3 were added to the medium dependent on which molecule was functionalized on the surface. TGFβ-1 was not included in the medium for a TGFβ-1 gradient*/*surface and TGFβ-3 was not included in the medium for a TGFβ-3 gradient*/*surface. Both TGFβ-1 and TGFβ-3 were added in the medium for GDF5 gradients*/*surfaces.* | | |

### Results

### Laminin control gradient surfaces

Laminin gradient surfaces (without morphogens) were used as controls to compare against the cellular responses from the morphogen gradient surfaces and h.d. surfaces. Cells on all replicated laminin gradient surfaces resulted in formation of condensed semi-circular structures consisting of cell free cavities, as shown in areas A1 and A2 in **Fig. 3**.

### GDF5 gradients and h.d. surfaces

Compared to results on laminin control gradient surfaces, c-iPSCs differentiated on GFD5 gradient surfaces resulted in another type of cell behaviour, condensed cell clusters/aggregates (termed *budding zones*), at a particle density of 500 to 1500 particles/µm² **(****Fig. 4****),** determined using SEM **(****Figs 1a** and **1b**). Larger cell masses were observed over the entire surface, but in the budding zone, small cell budding clusters were formed.

The differentiation process of iPSCs into chondrocytes is initiated by mesenchymal condensation, meaning reduction of intercellular spaces and is followed by continued differentiation. Cellular condensation is an important part of the differentiation process during chondrogenesis and skeletogenesis. To achieve a condensation process, the composition of the cellular microenvironment is crucial (Tacchetti, Tavella et al. 1992).

New homogenous density surfaces (h.d. surface) with a particle density in the range of the budding zone (500 to 1500 particles/µm²) was used to create surfaces with a homogenous GDF5 density **(****Fig. 5). Fig. 5A** shows a GDF5 h.d. surface with a particle density at 400 particles/µm², i.e., lower than the particle density at the observed budding zone. As seen in **Fig. 5B****,** the same cell budding phenomena as observed on the gradient surfaces was also observed on an h.d. surface having a particle density of 900 particles/µm². For comparison, on the h.d. surfaces with the lower particle density (400 particles/µm²) (**Fig. 5A**), cells formed larger gatherings as well but no indications of budding formations.

### Conclusion

The result found for the GDF5 gradient surfaces was especially noteworthy, where cell budding zones were observed in a specific density range (500 to 1500 particles/µm²), implicating the role of GDF5 at a certain concentration in a condensing budding formation. As described above, such condensation and budding formation is an indicative factor, though not confirmative, of that chondrogenesis has been induced.

The results were affirmed and repeated on specific h.d. surfaces where the cells behaved in the same way as observed on gradient surfaces, generating budding areas and a condensed structure. The appearance of cell clusters on both GDF5 gradient surfaces and h.d. surfaces provide important information for inducing the condensation and budding formation in chondrocyte differentiation, indicating an early stage in the formation of condensed chondrogenic structures in c-iPSCs. These results suggest that the cell budding responses are dependent on specific GDF5 concentrations and that the particular density interval was essential to induce cellular clusters.

### TGFβ-1 gradients and h.d. surfaces

c-iPSCs on all replicated TGFβ-1 gradient surfaces formed semi-circular structures in areas where the particle density was low, a behaviour comparable to control gradient surfaces with only laminin. At higher TGFβ-1 densities, this cell response was not observed; the cell development stimulated by TGFβ-1 resulted in evenly distributed cells. The cell responses were studied further on h.d. surfaces without a gradient, where separate surfaces with low and high particle density were used to compare the cellular responses when exposed to different single morphogen densities. At low TGFβ-1 h.d. surfaces (600 particles/µm²), cells tended to form the mentioned semi-circular structures consisting of cell free cavities covering the h.d. surfaces **(****Fig. 5C****).** At high TGFβ-1 h.d. surfaces (2000 particles/µm²), the cells were evenly distributed over the h.d. surfaces **(****Fig. 5D****).**

### Conclusion

It has been suggested that TGFβ, as well as GDF5, induce condensation (Wang, Rigueur et al. 2014). Surprisingly, c-iPSCs grown on TGFβ-1 gradient surfaces did not show condensational tendencies and did not respond similar to cells stimulated with GDF5 as no budding zone was observed on the TGFβ-1 gradient surfaces. Since TGFβ-1 is suggested as relevant to condensation specifically, the lack of budding was somewhat surprising.

At lower TGFβ-1 densities along the gradient surface, cells tended to form another structure in the shape of smaller clusters in a semicircular shape. However, this is more likely due to the effect of laminin. The low h.d. surfaces (600 particles/µm²) resulted, as expected from results from gradient surfaces, in that cells formed cell-free cavities, while cells on high h.d. surfaces (2000 particles/µm²) were evenly distributed, and no cavities were formed.

As cells on all replicated laminin gradient surfaces induced the same pattern seen at lower TGFβ-1 densities, it is most likely that low TGFβ-1 densities are not enough to override the effect of laminin. At higher densities, the effect of TGFβ-1 takes over and the cell uniformity is evident.

### TGFβ-3 gradients and h.d. surfaces

c-iPSCs on TGFβ-3 gradient surfaces did not generate any visual condensing cell response, i.e., the induced cell development resulted in evenly distributed cells. To compare and confirm the results, h.d. surfaces were used at high and low morphogen densities. Cells were evenly distributed over the gradient surfaces as well as the h.d. surfaces throughout the differentiation both at low (600 particles/µm²) and high (1900 particles/µm²) density (Fig. 5E and5F). No budding zone was observed and no tendency to otherwise condense; the cells were evenly distributed at microscopic and ocular inspection.

### Conclusion

Results from gradient surfaces with TGFβ-3 did not show any indications of budding formation or condensed structures. Thus, the conclusion is that TGFβ-3 is not as relevant as GDF5 for condensation and to generate budding zones, though being important for the cell development and differentiation.

As shown in **Figs. 5E** and **5F****,** the h.d. surfaces with a high (1900 particles/µm²) and a low (600 particles/µm²) particle density showed no specific cell responses such as observed primarily for GDF5, but also for TGFβ-1. In comparison to TGFβ-1, low densities of TGFβ-3 were not overridden by laminin and no cavities were formed, thus confirming TGFβ-3 to be a stronger influence than TGFβ-1 in this setting.

Overall, it may be concluded that no budding formation was observed for either TGFβ-1 or TGFβ-3, and when comparing the three different proteins, (GDF5, TGFβ-1, and TGFβ-3) it was clear that GDF5 induced condensation of cells to a higher extent than TGFβ-1 and TGFβ-3.

GDF5 and TGFβ-1 are both molecules that are involved in cellular condensation (Francis-West, Abdelfattah et al. 1999, Coleman, Vaughan et al. 2013; Kim et al. 2014). However, only on the GDF5 surfaces cells formed condensed budding structures. TGFβ-3 have shown to inhibit cellular condensation, which may correlate to the results in our in vitro setting (Jin et al. 2010). Thus, the similarities between TGFβ-1 and TGFβ-3 were somewhat surprising as well as the lack of similarities between the results for TGFβ-1 and GDF5.

### Immunostaining on c-iPSC seeded gradient surfaces and histological pellet sections

### Method

c-iPSCs on GDF5 gradient surfaces were stained for the proteins SOX9 and TBX3. Histological pellet sections where stained for TBX3. Also, TGFβ-3 h.d. surfaces were used as controls, as TGFβ-3 is known to provide increased aggrecan expression. A differentiation medium comprising GDF5 was added to the TGFβ-3 h.d. control surfaces to verify the importance of the surface densities of GDF5 and not only the presence of GDF5 *per se.* Paraffin embedded histological sections were deparaffinised and dehydrated prior immunostaining. Cells on surfaces and in pellets sections were permeabilised prior to immunostaining using 0.1% tritonX-100 (TritonX-100, Sigma Aldrich, USA) in PBS and incubated in room temperature for 10 minutes.

After permeabilisation, cells were incubated in blocking medium (2% BSA (Bovine Serum Albumin, Sigma Aldrich, USA), 0.1% tritonX-100, 100 mM glycine (Glycin, Riedel-de Haen AG, Germany) dissolved in PBS) for 15 minutes, followed by incubation in 500 µl of diluted specific primary antibody (Table 2) at 4°C overnight.

The next day, cells were rinsed and washed with PBS three times for 3 minutes. Thereafter, cells were incubated with blocking medium for 15 minutes followed by incubation with a secondary antibody for 2 hours in a humidity chamber (Table 2). After three rinsing/washing steps in PBS, each for 3 minutes, the cells and pellet sections were mounted with ProLong^{™} Gold Antifade Mountant media (ProLong^{™} Gold Antifade Mountant with DAPI, Thermo Fisher Scientific, USA) and were imaged using fluorescence microscopy (Eclipse 90i, Nikon Instruments, Japan) and confocal microscopy (Nikon A1, Nikon Instruments, Japan).

### Results

The c-iPSC behaviour resulting in a budding zone, found solely on GDF5 coated surfaces, were further studied using immunostaining for specific protein expressions, as described in the method section above.

Transcription factor SOX9 (SOX9) is well known to be an important regulating protein in chondrocyte differentiation, mesenchymal condensations and for formation and secretion of cartilage structural proteins. SOX9 is expressed in chondroprogenitors and differentiated chondrocytes but not in hypertrophic chondrocytes, indicating its importance in early cartilage development (Hino, Saito et al. 2014, Lefebvre and Dvir-Ginzberg 2017). Mutations in the SOX9 gene that leads to altered levels of SOX9 proteins is suggested to be linked to skeletal- and degeneration diseases (Lefebvre and Dvir-Ginzberg 2017). SOX9 is a chondrogenic marker and is therefore expected to be present during differentiation (Hino, Saito et al. 2014, Lefebvre and Dvir-Ginzberg 2017).

T-box transcription factor 3 (TBX3) is considered important for, and significantly expressed during, limb formation in early development (Sheeba and Logan 2017). Mutations, which have resulted in decreased protein expressions, are found to cause developmental defects such as ulnar-mammary syndrome (Bamshad, Le et al. 1999).

Hence, the presence of SOX9 and TBX3 are of high relevance when determining if differentiation of stem cells into chondrocytes have taken place.

Immunostaining using selected antibodies (Table 2) visualized expression of SOX9 and TBX3 respectively, in c-iPSCs differentiated on GDF5 gradient surfaces. All cell aggregates contained high SOX9 expressions **(****Fig. 6****).**

TBX3 expression was indeed observed to be specifically expressed in the buds found in the budding zone (500 to 1500 particles/µm²), primarily in the nuclei but also in forms of highly stained vesicles around nuclei **(****Fig. 7****).**

To validate these elevated levels of TBX3 observed in buds on GDF5 gradient surfaces, h.d. surfaces with a particle density corresponding to the budding zone were used.

For comparison, h.d. surfaces with GDF5 at low particle density (i.e., lower than the particle density within the GDF5 budding zone - 400 particles/µm²), TGFβ-3 (at the same particle density as within the GDF5 budding zone - 500 particles/µm²), TGFβ-3 surfaces (at the same particle density as within the GDF5 budding zone- 500 particles/µm²) with GDF5 added to the medium, and finally, glass slides coated with Coat-1 without added biomolecules were used as controls.

It was found that the TBX3 expression in cells differentiated on TGFβ-3 surfaces was more upregulated compared to all other controls, but still less than results from GDF5 surfaces. This finding confirms that TBX3 is more expressed at the GDF5 levels of the budding zone, as shown in **Fig. 8****.**

Cell condensation is seen to some extent **(****Fig. 8****),** but not in the way that budding zones are observed on the GDF5 gradient or h.d. surfaces within the budding zone range (500 to 1500 particles/µm²).

c-iPSC histological pellets were also stained for TBX3 using immuno-histochemistry **(****Fig. 9** **A3-6, B3, C3**). TBX3 were specifically induced in the area of the pellets with upregulated amounts of GAG, i.e., the induction of TBX3 could be specifically correlated to expression of GAG and was not correlated with the generally elevated expression of collagens.

**Table 2 - Antibodies and isotype control antibodies²**

| **Target** | **Primary antibody** | **Isotype control** | **Secondary antibody** |
|---|---|---|---|
| **TBX3** | Anti-TBX3 Antibody, LS-C133955, Nordic Biosite | Mouse IgG1 kappa Isotype Control, 14-4714-82, eBioscience, USA | Alexa Fluor 546, Invitrogen A11030, ThermoFisher Scientific, USA |
| **TBX3** | TBX3 polyclonal antibody, Invitrogen 42-4800, ThermoFisher Scientific, USA | | Alexa Fluor 594, Invitrogen A-21207, ThermoFisher Scientific, USA |
| **SOX9** | Anti-SOX9, ab76997, Abcam, United Kingdom | normal mouse IgG, sc-2025, Santa Cruz, USA | Alexa Fluor 546, Invitrogen A21133, ThermoFisher Scientific, USA |

| | | | |
|---|---|---|---|
| *²Antibodies and isotype control antibodies used for immunostaining of cells on GDF5 gradients.* | | | |

### Pellet cultures from GDF5 gradient surfaces, GDF5 h.d. surface and chondrocyte control experiments

### Method - GDF5 surface pellets and Chondrocyte pellet control experiments

The method described below was conducted using:
- GDF5 gradient surfaces seeded with c-iPSCs;
- GDF5 h.d. surfaces seeded with c-iPSCs - as control, having nanoparticles in a particle density of 440 particles/µm²;
- GDF5 h.d. surfaces seeded with c-iPSCs - having nanoparticles in a particle density within budding zone range. Such surface had particle densities as disclosed in the section *"Preparation of gradient surfaces of GDF5, TGFβ-1 and TGFβ-3* ".

After five days of differentiation on GDF5 gradient surfaces or h.d. surfaces, c-iPSCs were removed from each respective surface using trypsin-EDTA (0.05%) (ThermoFisher Scientific, USA).

The trypsin-EDTA reaction was quenched with human serum and the cells were transferred into 15ml conical tubes and centrifuged for 5 minutes at 1200g in 2ml differentiation medium with addition of both TGFβ-1 and TGFβ-3 (Table 1), to form a three-dimensional (3D) structure (also referred to as a "*pellet*" herein), which were to be further differentiated in a differentiation medium.

Said pellets were then stored in 96 well plates in 200µl differentiation medium (Table 1) in a 37°C incubator with 5% CO₂ and were differentiated for five weeks. The medium was changed every third day.

To compare results from the pellets with differentiated c-iPSC derived from GDF5 gradient surfaces and GDF5 h.d. surfaces (using the method presented above), two control setups were employed. The aim of the control setups was to compare the results from the three pellets with differentiated c-iPSC with pellets derived from healthy actual chondrocytes to verify that chondrocytes derived from c-iPSC behaved in the same way as ordinary, patient-derived chondrocytes, as the observed expression of SOX9 and TBX3 is not sufficient to conclude this, although being strongly indicative.

Chondrocytes cultured as described in the paragraph "Cell culturing of chondrocytes for control experiments" were used in the two control setups. In the first control experiment, chondrocytes were seeded to a GDF5 gradient surface and later formed into a pellet with the method described above. In the second control, chondrocytes were formed through a conventional pellet culture method not using a surface or gradient surface but with 5% human serum added to the differentiation medium. Pellets from the two controls were formed using the method described above.

Chondrocyte pellets were differentiated for two weeks since it has been shown that after two weeks, chondrocytes from OA donors have started to express high levels of both collagen type II and proteoglycans (Tallheden, Bengtsson et al. 2005). After the differentiation period, all pellets were fixated with HistofixTM (Histolab Products AB, Sweden) overnight and then stored in 70% ethanol.

Thereafter, all pellets were sent to HistoCenter (Mölndal, Sweden) for sectioning and staining with Alcian Blue van Gieson to visualize the extracellular matrix (ECM) composition of the pellets. The pellet compositions were analysed using a light microscope (Eclipse 90i, Nikon Instruments, Japan). Histological sections were stained with Alcian Blue van Gieson and displayed areas with a high amount of both blue colored glycosaminoglycans (GAGs) and red/purple coloured collagens.

### Results

As described in the method section above, cells were differentiated on GDF5 gradient surfaces and GDF5 h.d. surfaces (one particle density from the range of the budding zone, 500 to 1500 particles/µm², and one density with a lower particle density as a control, 400 particles/µm²) before being cultured as 3D micromass pellets suspended in differentiation medium (Table 1). A control having a particle density higher than 1500 particles/µm² was not tested partly due to that at higher concentrations than 1500, cells have a tendency to come off the substrate. As can be seen in **Fig. 4****,** cell growth is present also at higher particle densities, however, no budding formation is observed. Hence, on an h.d. surface, cells have difficulty to attach to the surface as a result of the higher particle densities causing such reference/control specimen to be unfit for control experiments.

The blue colour was mainly concentrated to one area of the pellets while the red/purple was expressed around the blue area and mostly in the distinct edges.

Two different chondrocyte pellets were used as controls. One using a GDF5 gradient surface and the same differentiation medium as the c-iPSC pellets (Table 1) **(****Fig. 9** **D1** & **D2)** and the other without GDF5 gradient or h.d. surface but the addition of 5% human serum to the medium **(****Fig. 9** **E1** & **E2).** Both chondrocyte control pellets seem to, compared to the c-iPSC pellets, have a more compact pellet structure. However, according to the colouring, the structural components in all pellets are similar.

The coloring of the c-iPSC pellets obtained from GDF5 gradient surfaces and h.d. surfaces were compared to chondrocyte control pellets and all indicated the presence of both GAGs (blue) and collagens (red/purple). This implies that the differentiation of the c-iPSC pellets using the protocol including GDF5 gradients and surfaces drives the cells meritoriously towards the chondrocyte linage.

Further, antibody staining (primary antibody collagen II; MP Biomedicals) also showed that differentiation of the c-iPSC using the protocol including GDF5 gradients (cf. Fig. 10A) results in expression of type II collagen, similar to chondrocytes maintained in serum (cf. Fig. 10B). Interestingly, chondrocytes maintained in the absence of serum, but first differentiated on a GDF5 gradient, essentially maintained the ability to express type II collagen (Fig. 10C). This ability was however lost in chondrocytes maintained also in the absence of serum, but first differentiated on a TGFβ-3 gradient (Fig. 10D), further indicating the need of GDF5 to drive the cells towards cartilage and not e.g., bone tissue.

### References

Aumailley, M. (2013). "The laminin family." Cell Adh Migr 7(1): 48-55.

Bamshad, M., T. Le, W. S. Watkins, M. E. Dixon, B. E. Kramer, A. D. Roeder, J. C. Carey, S. Root, A. Schinzel, L. Van Maldergem, R. J. Gardner, R. C. Lin, C. E. Seidman, J. G. Seidman, R. Wallerstein, E. Moran, R. Sutphen, C. E. Campbell and L. B. Jorde (1999). "The spectrum of mutations in TBX3: Genotype/Phenotype relationship in ulnar-mammary syndrome." Am J Hum Genet 64(6): 1550-1562.

Borestrom, C., S. Simonsson, L. Enochson, N. Bigdeli, C. Brantsing, C. Ellerstrom, J. Hyllner and A. Lindahl (2014). "Footprint-free human induced pluripotent stem cells from articular cartilage with redifferentiation capacity: a first step toward a clinical-grade cell source." Stem Cells Transl Med 3(4): 433-447.

Coleman, C. M., E. E. Vaughan, D. C. Browe, E. Mooney, L. Howard and F. Barry (2013). "Growth differentiation factor-5 enhances in vitro mesenchymal stromal cell chondrogenesis and hypertrophy." Stem Cells Dev 22(13): 1968-1976.

Francis-West, P. H., A. Abdelfattah, P. Chen, C. Allen, J. Parish, R. Ladher, S. Allen, S. MacPherson, F. P. Luyten and C. W. Archer (1999). "Mechanisms of GDF-5 action during skeletal development." Development 126(6): 1305-1315.

Goldring M B. Chondrogenesis, chondrocyte differentiation and articular cartilage metabolism in health and osteoarthrosis. Therapeutic Advances in Musculoskeletal Disease. 2012 Aug; 4(4): 269-285.

Hino, K., A. Saito, M. Kido, S. Kanemoto, R. Asada, T. Takai, M. Cui, X. Cui and K. Imaizumi (2014). "Master regulator for chondrogenesis, Sox9, regulates transcriptional activation of the endoplasmic reticulum stress transducer BBF2H7/CREB3L2 in chondrocytes." J Biol Chem 289(20): 13810-13820.

Kim Y I. Ryu J., Yeo J E., Choi Y J., Kim Y S., Ko K. and Koh Y. Overexpression of TGF-β1 enhances chondrogenic differentiation and proliferation of human synovium-derived stem cells. Biochemical and Biophysical Research Communications. Volume 450, Issue 4, 8 August 2014, Pages 1593-1599.

Lefebvre, V. and M. Dvir-Ginzberg (2017). "SOX9 and the many facets of its regulation in the chondrocyte lineage." Connect Tissue Res 58(1): 2-14.

Nguyen, D., D. A. Hagg, A. Forsman, J. Ekholm, P. Nimkingratana, C. Brantsing, T. Kalogeropoulos, S. Zaunz, S. Concaro, M. Brittberg, A. Lindahl, P. Gatenholm, A. Enejder and S. Simonsson (2017). "Cartilage Tissue Engineering by the 3D Bioprinting of iPS Cells in a Nanocellulose/Alginate Bioink." Sci Rep 7(1): 658.

Jin E J., Park K S., Kim D., Lee Y S., Sonn J., Chang J., Bang O., and Kang, S S., TGF-beta 3 inhibits chondrogenesis by suppressing precartilage condensation through stimulation of N-cadherin shedding and reduction of cRREB-1 expression. Molecules and cells. 29. 2010. 425-32.

Sheeba, C. J. and M. P. Logan (2017). "The Roles of T-Box Genes in Vertebrate Limb Development." Curr Top Dev Biol 122: 355-381.

Tacchetti, C., S. Tavella, B. Dozin, R. Quarto, G. Robino and R. Cancedda (1992). "Cell condensation in chondrogenic differentiation." Exp Cell Res 200(1): 26-33.

Tallheden T., Bengtsson C., Brantsing C., Sjögren-Jansson E., Carlsson L., Peterson L., Brittberg M., and Lindahl A. Proliferation and differentiation potential of chondrocytes from osteoarthritic patients. Arthritis Research & Therapy. 2005; 7(3): R560-R568.

Wang, W., D. Rigueur and K. M. Lyons (2014). "TGFbeta signaling in cartilage development and maintenance." Birth Defects Res C Embryo Today 102(1): 37-51.

## Claims

1. A method for differentiating induced pluripotent stem cells (iPSCs) into chondrocytes and integrating them into a matrix/scaffold to provide a cartilage implant, said method comprising the steps of:
- seeding a surface (110) of a substrate (100) with iPSCs, wherein the surface (110) is coated with nanoparticles (20) in a particle density of at least 500 particles/µm² and less than 1500 particles/µm², and parts (120) of the surface (110) in between said nanoparticles (20) are coated with a coating agent (40), wherein growth differentiation factor 5 (GDF5) molecules are attached to said nanoparticles (20);
- adding a first differentiation medium to the seeded iPSCs;
- allowing the seeded iPSCs to differentiate at least into chondrocyte progenitor cells on the surface (110) in the presence of the first differentiation medium; and
- integrating the obtained differentiated cells into a matrix/scaffold.

2. The method according to claim 1, wherein said iPSCs are differentiated for between 3 and 10 days, preferably between 4 and 8 days, on said substrate surface (110).

3. The method according to any one of claims 1 to 2, wherein the method further comprises:
- removing formed condensed chondrocyte progenitor cell aggregates from the substrate surface (110) with GDF5 attached thereto; and
- further differentiating the removed condensed chondrocyte progenitor cell aggregates into chondrocytes, in a second differentiation medium, wherein said removed condensed cell aggregates optionally are formed into a three-dimensional pellet structure, before being further differentiated; preferably the further differentiating of the removed condensed chondrocyte progenitor cell aggregates being performed for 4 to 10 weeks, such as for 5 to 8 weeks.

4. The method according to claims 3, wherein said first and second differentiation medium both comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof; and/or
wherein a maintenance medium is added to the substrate surface (110) after the iPSCs have been seeded to the surface (110) and before the differentiation medium is added.

5. The method according to any one of the preceding claims, wherein said iPSCs are chondrocyte derived iPSCs; preferably the chondrocyte derived iPSCs being generated by reprogramming chondrocytes using a method based on mRNA delivery; preferably the method being footprint-free.

6. The method according to any one of the preceding claims, wherein said coating agent (40) is a proliferative agent, preferably the proliferative agent being an extra cellular matrix (ECM) protein, such as a laminin or fibronectin, a cell specific cytokine or a combination thereof; and/or
wherein the nanoparticles (20) are gold particles coated with thiolated streptavidin, and wherein the GDF5 molecules are biotinylated and attached to the nanoparticles through biotin/streptavidin interaction.

7. The method according to any one of the preceding claims, wherein 20 000 to 100 000 iPSCs/cm², preferably 30 000 to 70 000 iPSCs/cm², more preferred about 50 000 iPSCs/cm², are seeded on the substrate surface (110).

8. A cartilage implant, wherein said cartilage implant is obtainable by differentiating induced pluripotent stem cells (iPSCs) into chondrocytes and integrating them into a matrix/scaffold in accordance with any one of claims 1 to 7.

9. A chondrocyte for use in chondrocyte implantation, said chondrocyte being integrated into a matrix/scaffold, wherein said chondrocyte is obtained by a method for differentiating induced pluripotent stem cells (iPSCs) into chondrocytes, said method comprising the steps of:
- seeding a surface (110) of a substrate (100) with iPSCs, wherein the surface (110) is coated with nanoparticles (20) in a particle density of at least 500 particles/µm² and less than 1500 particles/µm², and parts (120) of the surface (110) in between said nanoparticles (20) are coated with a coating agent (40), wherein growth differentiation factor 5 (GDF5) molecules are attached to said nanoparticles (20);
- adding a first differentiation medium to the seeded iPSCs;
- allowing the seeded iPSCs to differentiate at least into chondrocyte progenitor cells on the surface (110) in the presence of the first differentiation medium;
- removing formed condensed chondrocyte progenitor cell aggregates from the substrate surface (110) with GDF5 attached thereto; and
- further differentiating the removed condensed chondrocyte progenitor cell aggregates into chondrocytes, in a second differentiation medium.

10. The chondrocyte for use according to claim 9, wherein said iPSCs are differentiated for between 3 and 10 days, preferably between 4 and 8 days, on said substrate surface (110); and/or
wherein said removed condensed cell aggregates are formed into a three dimensional pellet structure, before being further differentiated.

11. The chondrocyte for use according to claim 9 to 10, wherein the step of further differentiating the removed condensed chondrocyte progenitor cell aggregates is performed for 4 to 10 weeks, preferably for 5 to 8 weeks.

12. The chondrocyte for use according to any one of the claims 9 to 11, wherein said first and second differentiation medium both comprise Dulbecco's modified Eagle's medium (DMEM), Insulin-Transferrin-Selenium, Ascorbic acid, Dexamethasone, Linoleic acid, Sodium Pyruvate, transforming growth factor beta 1 (TGFβ-1), transforming growth factor beta 3 (TGFβ-3), or a combination thereof; and/or
wherein a maintenance medium is added to the substrate surface (110) after the iPSCs have been seeded to the surface (110) and before the differentiation medium is added.

13. The chondrocyte for use according to any one of the claims 9 to 12, wherein said iPSCs are chondrocyte derived PSCs; preferably the chondrocyte derived PSCs being generated by reprogramming chondrocytes using a method based on mRNA delivery; preferably the method being footprint-free.

14. The chondrocyte for use according to any one of the claims 9 to 13, wherein
said coating agent (40) is a proliferative agent, preferably the proliferative agent being an extra cellular matrix (ECM) protein, such as a laminin or fibronectin, a cell specific cytokine or a combination thereof; and/or
wherein the nanoparticles (20) are gold particles coated with thiolated streptavidin, and wherein the GDF5 molecules are biotinylated and attached to the nanoparticles through biotin/streptavidin interaction; and/or
wherein 20 000 to 100 000 iPS cells/cm², preferably 30 000 to 70 000 iPS cells/cm², more preferred about 50 000 cells/cm², are seeded on the substrate surface (110).

15. The chondrocyte for use according to any one of the claims 9 to 14, further comprising a step of integrating the obtained chondrocytes into a matrix/scaffold.

## Patentansprüche

1. Verfahren zum Differenzieren induzierter pluripotenter Stammzellen (iPSCs) in Chondrozyten und zu deren Integration in eine Matrix/Gerüst, um ein Knorpelimplantat bereitzustellen, wobei das Verfahren die folgenden Schritte aufweist:
- Aussäen von iPSCs auf einer Oberfläche (110) eines Substrats (100), wobei die Oberfläche (110) mit Nanopartikeln (20) in einer Partikeldichte von mindestens 500 Partikel/µm² und weniger als 1500 Partikel/µm² beschichtet ist, und Teile (120) der Oberfläche (110) zwischen den Nanopartikeln (20) mit einem Beschichtungsmittel (40) beschichtet sind, wobei Wachstumsdifferenzierungsfaktor 5 (GDF5)-Moleküle an die Nanopartikel (20) gebunden sind;
- Zugeben eines ersten Differenzierungsmediums zu den ausgesäten iPSCs;
- Ermöglichen des Differenzierens der ausgesäten iPSCs zumindest in Chondrozyten-Vorläuferzellen auf der Oberfläche (110) in Gegenwart des ersten Differenzierungsmediums; und
- Integrieren der erhaltenen differenzierten Zellen in eine Matrix/ein Gerüst.

2. Verfahren nach Anspruch 1, wobei die iPSCs zwischen 3 und 10 Tagen, vorzugsweise zwischen 4 und 8 Tagen, auf der Substratoberfläche (110) differenziert werden.

3. Verfahren nach einem der Ansprüche 1 bis 2, wobei das Verfahren ferner aufweist:
- Entfernen von gebildeten kondensierten Chondrozyten-Vorläuferzellaggregaten von der Substratoberfläche (110) mit daran gebundenem GDF5; und
- weiter Differenzieren der entfernten kondensierten Chondrozyten-Vorläuferzellaggregate in Chondrozyten in einem zweiten Differenzierungsmedium, wobei die entfernten kondensierten Zellaggregate gegebenenfalls zu einer dreidimensionalen Pelletstruktur geformt werden, bevor sie weiter differenziert werden; wobei vorzugsweise das weitere Differenzieren der entfernten kondensierten Chondrozyten-Vorläuferzellaggregate für 4 bis 10 Wochen, beispielsweise für 5 bis 8 Wochen, ausgeführt wird.

4. Verfahren nach Anspruch 3, wobei das erste und das zweite Differenzierungsmedium beide Dulbeccos Modifikation des Eagle Mediums (DMEM), Insulin-Transferrin-Selen, Ascorbinsäure, Dexamethason, Linolsäure, Natriumpyruvat, transformierenden Wachstumsfaktor beta 1 (TGFβ-1), transformierenden Wachstumsfaktor beta 3 (TGFβ-3) oder eine Kombination davon aufweisen; und/oder
wobei der Substratoberfläche (110) ein Erhaltungsmedium zugesetzt wird, nachdem die iPSCs auf der Oberfläche (110) ausgesät worden sind und bevor das Differenzierungsmedium zugesetzt wird.

5. Verfahren nach einem der vorhergehenden Ansprüche, wobei die iPSCs von Chondrozyten abgeleitete iPSCs sind; wobei vorzugsweise die von Chondrozyten abgeleiteten iPSCs durch Reprogrammierung von Chondrozyten unter Verwendung eines auf mRNA-Bereitstellung basierenden Verfahrens erzeugt werden; wobei vorzugsweise das Verfahren footprintfrei-ist.

6. Verfahren gemäß einem der vorhergehenden Ansprüche, wobei das Beschichtungsmittel (40) ein Proliferationsmittel ist, wobei das Proliferationsmittel vorzugsweise ein extrazelluläres Matrix (ECM)-Protein, wie z.B. ein Laminin oder Fibronektin, ein zellspezifisches Zytokin oder eine Kombination davon ist; und/oder
wobei die Nanopartikel (20) Goldpartikel sind, die mit thioliertem Streptavidin beschichtet sind, und wobei die GDF5-Moleküle biotinyliert und durch Biotin/Streptavidin-Wechselwirkung an die Nanopartikel gebunden sind.

7. Verfahren nach einem der vorhergehenden Ansprüche, wobei 20 000 bis 100 000 iPSCs/cm², vorzugsweise 30 000 bis 70 000 iPSCs/cm², noch weiter bevorzugt etwa 50 000 iPSCs/cm², auf der Substratoberfläche (110) ausgesät werden.

8. Knorpelimplantat, wobei das Knorpelimplantat durch Differenzieren induzierter pluripotenter Stammzellen (iPSCs) zu Chondrozyten und deren Integrieren in eine Matrix/Gerüst nach einem der Ansprüche 1 bis 7 erhältlich ist.

9. Chondrozyt zur Verwendung bei der Chondrozyten-Implantation, wobei der Chondrozyt in eine Matrix/ein Gerüst integriert wird, wobei der Chondrozyt durch ein Verfahren zum Differenzieren induzierter pluripotenter Stammzellen (iPSCs) in Chondrozyten erhalten wird, wobei das Verfahren die folgenden Schritte aufweist:
- Aussäen von iPSCs auf eine Oberfläche (110) eines Substrats (100), wobei die Oberfläche (110) mit Nanopartikeln (20) in einer Partikeldichte von mindestens 500 Partikel/µm₂ und weniger als 1500 Partikel/µm² beschichtet ist, und Teile (120) der Oberfläche (110) zwischen den Nanopartikeln (20) mit einem Beschichtungsmittel (40) beschichtet sind, wobei Wachstumsdifferenzierungsfaktor 5 (GDF5)-Moleküle an die Nanopartikel (20) gebunden sind;
- Zugeben eines ersten Differenzierungsmediums zu den ausgesäten iPSCs;
- Ermöglichen des Differenzierens der ausgesäten iPSCs zumindest in Chondrozyten-Vorläuferzellen auf der Oberfläche (110) in Gegenwart des ersten Differenzierungsmediums;
- Entfernen von gebildeten kondensierten Chondrozyten-Vorläuferzellaggregaten von der Substratoberfläche (110) mit daran gebundenem GDF5; und
- weiter Differenzieren der entfernten kondensierten Chondrozyten-Vorläuferzellaggregate in Chondrozyten in einem zweiten Differenzierungsmedium.

10. Chondrozyt zur Verwendung nach Anspruch 9, wobei die iPSCs zwischen 3 und 10 Tagen, vorzugsweise zwischen 4 und 8 Tagen, auf der Substratoberfläche (110) differenziert werden; und/oder
wobei die entfernten kondensierten Zellaggregate zu einer dreidimensionalen Pelletstruktur geformt werden, bevor sie weiter differenziert werden.

11. Chondrozyt zur Verwendung nach einem der Ansprüche 9 bis 10, wobei der Schritt des weiteren Differenzierens der entfernten kondensierten Chondrozyten-Vorläuferzellaggregate für 4 bis 10 Wochen, vorzugsweise für 5 bis 8 Wochen, ausgeführt wird.

12. Chondrozyt zur Verwendung nach einem der Ansprüche 9 bis 11, wobei das erste und das zweite Differenzierungsmedium beide Dulbeccos Modifikation des Eagle Mediums (DMEM), Insulin-Transferrin-Selen, Ascorbinsäure, Dexamethason, Linolsäure, Natriumpyruvat, transformierenden Wachstumsfaktor beta 1 (TGFβ-1), transformierenden Wachstumsfaktor beta 3 (TGFβ-3) oder eine Kombination davon aufweisen; und/oder
wobei der Substratoberfläche (110) ein Erhaltungsmedium zugesetzt wird, nachdem die iPSCs auf der Oberfläche (110) ausgesät worden sind und bevor das Differenzierungsmedium zugesetzt wird.

13. Chondrozyt zur Verwendung nach einem der Ansprüche 9 bis 12, wobei die iPSCs von Chondrozyten abgeleitete PSCs sind; wobei vorzugsweise die von Chondrozyten abgeleiteten PSCs durch Reprogrammierung von Chondrozyten unter Verwendung eines auf mRNA-Bereitstellung basierenden Verfahrens erzeugt werden; wobei vorzugsweise das Verfahren footprintfrei ist.

14. Chondrozyt zur Verwendung nach einem der Ansprüche 9 bis 13, wobei
das Beschichtungsmittel (40) ein Proliferationsmittel ist, wobei das Proliferationsmittel vorzugsweise ein extrazelluläres Matrix (ECM)-Protein, wie z.B. ein Laminin oder Fibronektin, ein zellspezifisches Zytokin oder eine Kombination davon ist; und/oder
wobei die Nanopartikel (20) Goldpartikel sind, die mit thioliertem Streptavidin beschichtet sind, und wobei die GDF5-Moleküle biotinyliert sind und durch Biotin/Streptavidin-Wechselwirkung an die Nanopartikel gebunden sind; und/oder
wobei 20 000 bis 100 000 iPS-Zellen/cm², vorzugsweise 30 000 bis 70 000 iPS-Zellen/cm², noch weiter bevorzugt etwa 50 000 Zellen/cm², auf der Substratoberfläche (110) ausgesät werden.

15. Chondrozyt zur Verwendung nach einem der Ansprüche 9 bis 14, des Weiteren aufweisend einen Schritt des Integrierens der erhaltenen Chondrozyten in eine Matrix/Gerüst.

## Revendications

1. Procédé pour différencier des cellules-souches pluripotentes induites (iPSC) en chondrocytes et les intégrer dans une matrice/échafaudage pour fournir un implant cartilagineux, ledit procédé comprenant les étapes consistant à :
- ensemencer une surface (110) d'un substrat (100) avec des iPSC, dans lequel la surface (110) est revêtue de nanoparticules (20) selon une densité de particule d'au moins 500 particules/µm² et moins de 1500 particules/µm², et des parties (120) de la surface (110) entre lesdites nanoparticules (20) sont revêtues d'un agent de revêtement (40), dans lequel des molécules à facteur de différenciation de croissance 5 (GDF5) sont fixées auxdites nanoparticules (20) ;
- ajouter un premier milieu de différenciation aux iPSC ensemencées ;
- permettre aux iPSC ensemencées de se différencier au moins en cellules progénitrices de chondrocytes sur la surface (110) en présence du premier milieu de différenciation ; et
- intégrer des cellules différenciées obtenues dans une matrice/échafaudage.

2. Procédé selon la revendication 1, dans lequel lesdites iPSC sont différenciées pour une durée comprise entre 3 et 10 jours, de préférence entre 4 et 8 jours, sur ladite surface de substrat (110).

3. Procédé selon l'une quelconque des revendications 1 à 2, dans lequel le procédé comprend en outre :
- le retrait d'agrégats de cellules progénitrices de chondrocytes condensées formés de la surface de substrat (110) à laquelle sont fixées des molécules à GDF5 ; et
- la différenciation supplémentaire des agrégats de cellules progénitrices de chondrocytes condensées retirés en chondrocytes, dans un deuxième milieu de différenciation, dans lequel lesdits agrégats de cellules condensées retirés sont optionnellement formés dans une structure de granule en trois dimensions, avant d'être en outre différenciés ; de préférence la différenciation supplémentaire des agrégats de cellules progénitrices de chondrocytes condensées retirés étant réalisée pendant 4 à 10 semaines, tel que 5 à 8 semaines.

4. Procédé selon la revendication 3, dans lequel lesdits premier et deuxième milieux de différenciation comprennent tous les deux un milieu Eagle modifié de Dulbecco (DMEM), de l'insuline-transferrine-sélénium, de l'acide ascorbique, de la dexaméthasone, de l'acide linoléique, du pyruvate de sodium, un facteur de croissance transformant beta 1 (TGFβ-1), un facteur de croissance transformant beta 3 (TGFβ-3) ou une combinaison de ceux-ci ; et/ou
dans lequel un milieu de maintien est ajouté à la surface de substrat (110) après que les iPSC ont été ensemencées sur la surface (110) et avant l'ajout du milieu de différenciation.

5. Procédé selon l'une quelconque des revendications précédentes, dans lequel lesdites iPSC sont des iPSC dérivées de chondrocytes ; de préférence les iPSC dérivées de chondrocytes sont produites par la reprogrammation de chondrocytes à l'aide d'un procédé basé sur l'administration d'ARNm ; de préférence le procédé est sans empreinte.

6. Procédé selon l'une quelconque des revendications précédentes, dans lequel ledit agent de revêtement (40) est un agent de prolifération, de préférence l'agent de prolifération étant une protéine de matrice extracellulaire (ECM), telle qu'une laminine ou une fibronectine, une cytokine spécifique de la cellule ou une combinaison de celles-ci ; et/ou
dans lequel les nanoparticules (20) sont des particules d'or revêtues de streptavidine thiolée, et dans lequel les molécules de GDF5 sont biotinylées et fixées aux nanoparticules par le biais d'une interaction de biotine/streptavidine.

7. Procédé selon l'une quelconque des revendications précédentes, dans lequel 20 000 à 100 000 iPSC/cm², de préférence 30 000 à 70 000 iPSC/cm², plus préférablement environ 50 000 iPSC/cm², sont ensemencées sur la surface de substrat (110).

8. Implant cartilagineux, dans lequel ledit implant cartilagineux peut être obtenu en différenciant des cellules-souches pluripotentes induites (iPSC) en chondrocytes et en les intégrant dans une matrice/échafaudage conformément à l'une quelconque des revendications 1 à 7.

9. Chondrocyte pour utilisation dans une implantation de chondrocyte, ledit chondrocyte étant intégré dans une matrice/échafaudage, dans lequel ledit chondrocyte est obtenu par un procédé de différenciation de cellules-souches pluripotentes induites (iPSC) en chondrocytes, ledit procédé comprenant les étapes consistant à :
- ensemencer une surface (110) d'un substrat (100) avec des iPSC, dans lequel la surface (110) est revêtue de nanoparticules (20) selon une densité de particule d'au moins 500 particules/µm² et moins de 1500 particules/µm², et des parties (120) de la surface (110) entre lesdites nanoparticules (20) sont revêtues d'un agent de revêtement (40), dans lequel des molécules à facteur de différenciation de croissance 5 (GDF5) sont fixées auxdites nanoparticules (20) ;
- ajouter un premier milieu de différenciation aux iPSC ensemencées ;
- permettre aux iPSC ensemencées de se différencier au moins en cellules progénitrices de chondrocytes sur la surface (110) en présence du premier milieu de différenciation ;
- retirer des agrégats de cellules progénitrices de chondrocytes condensées formés de la surface de substrat (110) à laquelle sont fixées des molécules à GDF5 ; et
- différencier en outre des agrégats de cellules progénitrices de chondrocytes condensées retirés en chondrocytes, dans un deuxième milieu de différenciation.

10. Chondrocyte pour utilisation selon la revendication 9, dans lequel lesdites iPSC sont différenciées pendant entre 3 et 10 jours, de préférence entre 4 et 8 jours, sur ladite surface de substrat (110) ; et/ou
dans lequel lesdits agrégats de cellules condensées retirés sont formés dans une structure de granule en trois dimensions, avant d'être à nouveau différenciés.

11. Chondrocyte pour utilisation selon la revendication 9 à 10, dans lequel l'étape de différenciation supplémentaire des agrégats de cellules progénitrices de chondrocytes condensées retirés est réalisée pendant 4 à 10 semaines, de préférence pendant 5 à 8 semaines.

12. Chondrocyte pour utilisation selon l'une quelconque des revendications 9 à 11, dans lequel lesdits premier et deuxième milieux de différenciation comprennent tous les deux un milieu Eagle modifié de Dulbecco (DMEM), de l'insuline-transferrine-sélénium, de l'acide ascorbique, de la dexaméthasone, de l'acide linoléique, du pyruvate de sodium, un facteur de croissance transformant beta 1 (TGFβ-1), un facteur de croissance transformant beta 3 (TGFβ-3) ou une combinaison de ceux-ci ; et/ou
dans lequel un milieu de maintien est ajouté à la surface de substrat (110) après que les iPSC ont été ensemencées sur la surface (110) et avant l'ajout du milieu de différenciation.

13. Chondrocyte pour utilisation selon l'une quelconque des revendications 9 à 12, dans lequel lesdites iPSC sont des iPSC dérivées de chondrocytes ; de préférence les iPSC dérivées de chondrocytes sont produites par la reprogrammation de chondrocytes à l'aide d'un procédé basé sur l'administration d'ARNm ; de préférence le procédé est sans empreinte.

14. Chondrocyte pour utilisation selon l'une quelconque des revendications 9 à 13, dans lequel
ledit agent de revêtement (40) est un agent de prolifération, de préférence l'agent de prolifération étant une protéine de matrice extracellulaire (ECM), telle qu'une laminine ou une fibronectine, une cytokine spécifique de la cellule ou une combinaison de celles-ci ; et/ou
dans lequel les nanoparticules (20) sont des particules d'or revêtues de streptavidine thiolée, et dans lequel les molécules de GDF5 sont biotinylées et fixées aux nanoparticules par le biais d'une interaction de biotine/streptavidine ; et/ou
dans lequel 20 000 à 100 000 iPSC/cm², de préférence 30 000 à 70 000 iPSC/cm², plus préférablement environ 50 000 iPSC/cm², sont ensemencées sur la surface de substrat (110) .

15. Chondrocyte pour utilisation selon l'une quelconque des revendications 9 à 14, comprenant en outre une étape d'intégration des chondrocytes obtenus dans une matrice/échafaudage.
